# EUROPEAN PATENT APPLICATION

(11) **EP 2 557 079 A1**
(43) Date of publication of application: **13.02.2013**
(21) Application number: 11176996.4
(22) Date of filing: 09.08.2011
(51) Int. Cl.: C07D 311/62, C07H 15/26

(54) **Synthesis of catechin and epicatechin conjugates**

(71) Applicant: Nestec S.A., 1800 Vevey (CH)
(72) Inventor: Actis Goretta, Lucas, 1010 Lausanne (CH); Viton, Florian, 1000 Lausanne 26 (FR); Barron, Denis, 1095 Lutry (CH); Dionisi, Fabiola, 1066 Epalinges (CH)
(74) Representative: Marquardt, Ulf

(57) **Abstract**

The present invention relates generally to catechin and epicatechin conjugates of formula (I). For example, a chemical synthesis process for the preparation of catechin and epi-catechin compounds, in particular of catechin and epi-catechin conjugates is disclosed. A further aspect of the invention pertains to new catechin and epi-catechin conjugate compounds.

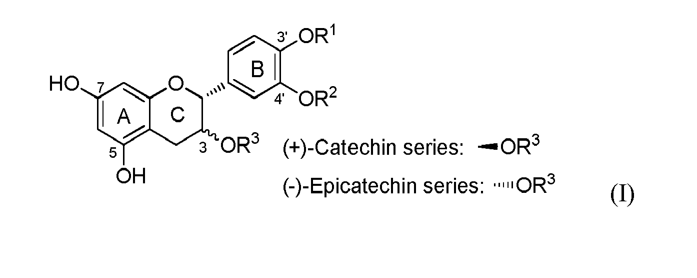

## Description

### Field of the invention

The present invention relates generally to catechin and epicatechin conjugates. For example, a chemical synthesis process for the preparation of catechin and epi-catechin compounds, in particular of catechin and epi-catechin conjugates is disclosed. A further aspect of the invention pertains to new catechin and epi-catechin conjugate compounds.

### Background of the invention

Flavonoids, also collectively known as "vitamin P" or citrin, are secondary metabolites of plants. One class of those flavonoids are the flavan-3-ols *(catechins* and *proanthocyanidins)* that use the 2-phenyl-3,4-dihydro-2H-chromen-3-ol as skeleton of their molecular structure. The flavan-3-ols are the most abundant polyphenolic compounds found in many plant materials used by humans and animals as foods that have been related to beneficial health effects. Indeed, a large number of epidemiological studies demonstrate that chronic consumption of flavanol-rich foods, as e.g. from fresh fruits, tea, cocoa and red wine, leads to a reduction of atherosclerotic plaques in animal models, and is inversely associated with the risk of coronary heart disease, certain types of cancer, and immunodysfunctions. These epidemiological observations are supported by numerous *in vitro* and *ex vivo* mechanistic studies. Thus, for example, reduction of carcinogenesis was seen *in vitro* (Mittal, A. et al., Int. J. Oncol. 2004, 24 (3), 703-710). Despite considerable research dedicated to understanding the flavanol-induced health promoting effects, including endothelial function, blood pressure, platelet activity and reduction of the oxidative stress, the precise biological targets of these compounds remain largely unknown.

There is an interest in the field to study and understand flavanol-induced biological effects in humans and animals, including the mechanism of absorption, metabolism in the body and secretion of these compounds. So far it is known that catechins are absorbed from the human intestinal tract at the site of enterocytes. Catechins are then rapidly metabolized at the site of their absorption and distributed as conjugated derivatives in blood. Physiological processing of catechins is then completed in the liver at the site of hepatocytes, and it is these conjugated forms that are excreted in urine. In both, the enterocytes and hepatocytes, the conjugation of catechins with hydrophilic moieties is catalyzed by phase II metabolism enzymes, including methyl-, glucuronyl-, and sulfo-transferases.

The actual flavanol metabolites and conjugates are likely to possess biological targets different from those of the parent flavan-3-ols. Therefore, it would be important to evaluate their activity, for which sufficient amounts are required that cannot be obtained by direct isolation from biological fluids such as blood or urine. A method for preparing the various catechin and epi-catechin conjugates in a regio-controlled manner would provide a material basis for studying the activity and distribution patterns of the metabolites in order to explain their role as actors in mediating the beneficial health effects associated with the intake of catechins and flavonoids in general.

Studies have been published regarding the synthesis and activity of conjugated metabolites of quercetin (Yoshizumi, M. et al., Biochem. Biophys. Res. Commun. 2002, 293 (5), 1458-1465). However, little data exists concerning catechin and epi-catechin metabolites. The different available methods for preparing catechin and epi-catechin conjugates can be partitioned into three categories, in terms of increasing regio-selectivity of the approach, respectively: (i) direct derivatization, (ii) hemi-synthetic or "hybrid" approach, and (iii) total synthesis.

The direct derivatization approach is exemplified by the works of Gonzalez-Manzano, S. et al. in J. Agric. Food Chem. 2009, 57 (4), 1231-1238. It involves a non-selective treatment of commercially available (-)-epicatechin with an excess of the conjugation reagent (i.e. glucuronidation, sulfation or methylation) followed by fractionation of the obtained regio-isomeric mixture by (semi)preparative HPLC techniques. The optimized procedure allowed preparation of (epi)catechin sulfates, glucuronides and methyl ethers conjugated at either position 3' or 4', as well as the sulfates at positions 5 or 7 for their further isolation by semi-preparative HPLC. The main advantage of this methodology includes the use of commercially available, optically pure (-)-epicatechin as starting material, thus requiring no *de novo* synthesis of the latter molecule. When employing this direct derivatization method, the conjugates are obtained in "one pot" and need to be separated from the complex isomeric mixture. Beside the very low yields of conjugation, the main disadvantage of the direct derivatization approach is the inherent lack of region-selectivity in the conjugation of the (epi)catechin. Furthermore, it is impossible to obtain composite conjugates because of the vanishingly low throughput of the first conjugation step. Further, the lack of the 4-carbonyl and the 2,3-double bond results in similar acidities for all the phenolic hydroxyls and therefore no region-selective conjugation can be achieved. The technique also suffers from low throughput, as large quantities of the (relatively expensive) starting material are required for the preparation of minute amounts of a single desired conjugated isomer. Furthermore, similar retention times of the variously substituted (epi)catechin metabolites further decrease isolation yields and also compromise the purity of the isolated compounds.

Incorporation of moderate levels of regio-selectivity into the direct derivatization process leads to the so-called hemi-synthetic or "hybrid" approach. This technology has been described by C. Rolando et al., J. Chem. Soc. , Perkin Trans. 1 2002, (6), 821-830, and applied by the same group to the regio-selective preparation of the four monomethylated isomers of (+)-catechin in positions 3', 4', 5 and 7, two dimethylated derivatives, the 5,7-dimethyl (+)-catechin and the 3',4'-dimethyl (+)-catechin and two trimethylated isomers of (+)-catechin in positions 3',5,7 and 4',5,7. The "hybrid" method incorporates some regio-chemistry and is based on successive and selective protections of the various phenol functions present on (+)-catechin. The key step of this hemi-synthesis is the differentiation of the catechol B ring of (+)-catechin from the phloroglucinol A ring by the formation of permanent or transient dioxolanes. Advantages of Rolando's technology are the regio-selectivity, the use of commercially available and optically pure (+)-catechin as starting material, and the few chemical steps required in the process. The main disadvantage of the "hybrid" technology is its inherent lack of modularity. Indeed, Rolando's methodology is limited to the hemi-synthesis of 'simple' (epi)catechin conjugates and cannot be applied to the preparation of composite (epi)catechin conjugates. Another disadvantage is the low yield of the selective protection of the (epi)catechin at the free catechol ring, i.e. the first step in the hemi-synthetic process.

While several stereo-selective syntheses of optically active (epi)catechin and derivatives have been reported in the literature (see e.g. Wan et al. Bioorg. Med. Chem. 2004, 12 (13), 3521-3527; Ding et al. Chin. J. Chem. 2006, 24 (11), 1618-1624; Wan et al. Bioorg. Med. Chem. 2005, 13 (6), 2177-2185; Jew et al. Tetrahedron: Asymmetry 2002, 13 (7), 715-720; Anderson et al. Tetrahedron 2005, 61 (32), 7703-7711; van Rensburg et al. J. Chem. Soc. , Perkin Trans. 1 1997, (22), 3415-3422), to our knowledge no such synthesis of the corresponding metabolites (methyl ethers, sulfates or glucuronides) has been reported this far. Particularly, none of the described synthetic pathways allowed differentiating the hydroxyl groups of the catechol B ring of the (epi)catechin framework from the hydroxyl groups of the phloroglucinol A ring, thereby not offering the possibility to introduce a conjugation at a specific site of the (epi)catechins in a regio-controlled manner.

Hence, there is a clear need in the art to find a way to chemically synthesize a variety of optically active catechin and epicatechin conjugate derivatives that are chemically pure and available in quantities in the milligram range to allow *in vitro* and *in vivo* experimentation.

### Summary of the invention

The object of the present invention is to provide a solution to the above cited need for a new chemical synthesis process and to provide novel optically active catechin and epicatechin conjugate derivatives.

The object of the present invention is achieved by the subject matter of the independent claims. The dependent claims further develop the idea of the present invention.

Accordingly, the present invention provides in a first aspect a process for the preparation of a compound of the general formula (I) 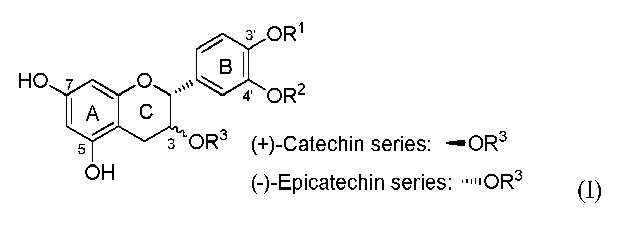 , wherein R¹ and R² are independently H, methyl, sulfonyl, glucuronyl or glucoside; and R³ is H or a gallate; which comprises
a) a coupling reaction step of a compound of formula (II) 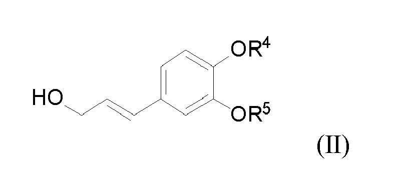 , wherein R⁴ and R⁵ are independently H, methyl or a protecting group; with a compound of formula (III) 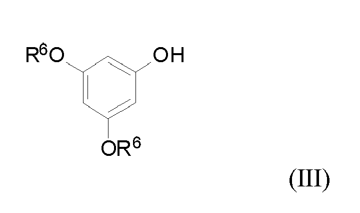 , wherein R⁶ is a protecting group; and
b) a stereo-selective intra-molecular trans-cyclization reaction step of a product further elaborated from the coupled product of step a).

In a second aspect, the invention relates to a compound of the general formula (I) 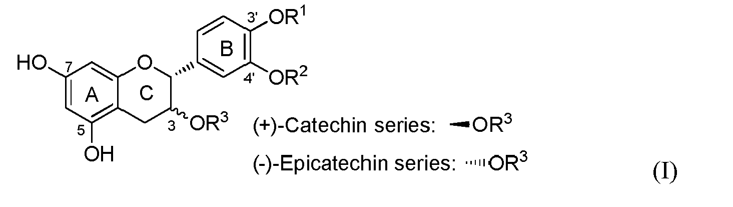 , wherein
R¹ and R² are independently methyl, sulfonyl, glucuronyl or glucoside, but are not both glucuronyl and/or glucoside; R³ is H or a gallate; and
wherein the compound is not 4'-*O*-methyl-(-)-epicatechin-3'-O-glucuronide.

A still further aspect of the invention is a food product comprising the compounds of the invention.

The inventors were surprised to find that when coupling first a compound of the general formula (II) with another compound of the general formula (III) as starting materials in a chemical synthesis, and then in a later step going through a stereo-selective intra-molecular trans-cyclization reaction, catechin and epi-catechin derivative compounds can be synthesized in substantially chemically pure form and in substantial quantities, for example in the [mg] range.

Advantageously, the combination in a first coupling reaction of the compound of formula (II) with the compound of formula (III) allows first to independently modulate and protect the prospective OH groups of the B and/or A ring of the catechin or epicatechin derivative molecules to be achieved before going through the coupling and trans-cyclization reaction which generates the actual catechin/epicatechin backbone structure. This offers a great chemical modularity. The total synthesis approach is unique in being able to provide access to all types of (epi)catechin metabolites (simple and composite) of remarkable purity and employing unexpensive "bulk" starting materials. The total synthesis approach of the present invention represents the most selective access to isomerically pure (epi)catechin conjugates.

A further advantage of the invention is that it provides a variety of novel flavan-3-ol conjugated derivative molecules, including methylated, sulfated, glucuronidated, glucosided and/or any combination molecules thereof. Such molecules can actually be found in minute amounts as circulating metabolites in body fluids of humans or animals, for example after immediate consumption of flavanol-rich foods, or have as of today not been identified or described in the literature yet. Such novel molecules can now advantageously be used for example as markers or standards in analytical experiments, as lead molecules in *in vitro* or *in vivo* experiments for elaborating molecular mechanisms of the effects of such molecules in an animal or human body, and/or as additives or ingredients in food products for humans and animals as functional molecules attaining certain health benefits to a consumer of such a food product. Such food products can also be parenteral solutions as for example for clinical health care nutrition with the objective to provide the health beneficial effects of those compounds directly to a body in need with bypassing the intestinal absorption barrier.

### Brief description of the drawings

Figure 1: Synthesis of (E)-2-(benzyloxy)-4-(3-hydroxyprop-1-en-1-yl)phenol
Figure 2: Synthesis of (E)-2-(benzyloxy)-5-(3-hydroxyprop-1-en-1-yl)phenol
Figure 3: Synthesis of (E)-4-(3-hydroxyprop-1-enyl)-2-methoxyphenol
Figure 4: Synthesis of 3,5-Di-O-Benzyl Phloroglucinol
Figure 5: Synthesis of (E)-3,5-bis(benzyloxy)-2-(3-(3-(benzyloxy)-4-hydroxyphenyl)allyl)phenol
Figure 6: Synthesis of (E)-3,5-bis(benzyloxy)-2-(3-(4-(benzyloxy)-3-hydroxyphenyl)allyl)phenol
Figure 7: Synthesis of (E)-3,5-bis(benzyloxy)-2-(3-(4-hydroxy-3-methoxyphenyl)allyl)phenol
Figure 8: Synthesis of (1S,2S)-1-(3-(benzyloxy)-4-((tert-butyldimethylsilyl)oxy)phenyl)-3-(2,4-bis(benzyloxy)-6-((tert-butyldimethylsilyl)oxy)phenyl)propane-1,2-diol
Figure 9: Synthesis of (1S,2S)-1-(4-(benzyloxy)-3-((tert-butyldimethylsilyl)oxy)phenyl)-3-(2,4-bis(benzyloxy)-6-((tert-butyldimethylsilyl)oxy)phenyl)propane-1,2-diol
Figure 10: Synthesis of (1S,2S)-3-(2,4-bis(benzyloxy)-6-((tert-butyldimethylsilyl)oxy)phenyl)-1-(4-((tert-butyldimethylsilyl)oxy)-3-methoxyphenyl)propane-1,2-diol
Figure 11: Synthesis of (2R,3S)-5,7-di-O-benzyl-3'-O-methyl 3-acetylcatechin
Figure 12: Synthesis of (2R,3S)-5,7,3'-tri-O-benzyl-3-acetylcatechin
Figure 13: Synthesis of (2R,3S)-5,7,4'-tri-O-benzyl-3-acetylcatechin
Figure 14: Synthesis of (2R,3R)-3'-(tert-butyldimethylsilyl)-5,7,4'-tri-O-benzyl-epi-catechin
Figure 15: Synthesis of (2R,3R)-4'-(tert-butyldimethylsilyl)-5,7-di-O-benzyl-3'-O-methyl epi-catechin
Figure 16: Synthesis of (2R,3S)-catechin 4'-β-D-glucuronic acid
Figure 17: Synthesis of (2R,3R)-epi-catechin 3'-β-D-glucuronic acid
Figure 18: Synthesis of (2R,3R)-3'-O-methyl epicatechin 4'-sodium sulfate
Figure 19: Synthesis of (2R,3R)-epi-catechin 3'-sodium sulfate

### Detailed description of the invention

The present invention pertains to the process for the preparation of a compound of the general formula (I), wherein R¹ and R² are independently H, methyl, sulfonyl, glucuronyl or glucoside; and R³ is H or a gallate; which comprises a) a coupling reaction step of a compound of formula (II), wherein R⁴ and R⁵ are independently H, methyl or a protecting group; with a compound of formula (III), wherein R⁶ is a protecting group; and b) a stereo-selective intra-molecular trans-cyclization reaction step of the coupled product of step a); wherein for the compound of the formula (II) R⁴ is not equal R⁵. This allows to make full use of the modularity of the inventive process and to produce catechin and epi-catechin molecules with different conjugates at position 3' and 4' at the B ring of a same final compound. So far, it was not possible to produce such composite conjugated compounds by chemical synthesis.

The process of the invention further comprises a Sharpless dihydroxylation reaction step after step a) and before step b). This reaction allows introduction of chirality at C3 in the flavan-3-ol framework with high optical purity. It can be performed according to Sharpless et al. (J. Org. Chem. 1992, 57 (10), 2768-2771 and Chem. Rev. (Washington, D. C. ) 1994, 94 (8), 2483-2547) with commercially available stereoselective catalysts AD-mix-α, or AD-mix-β.

In one embodiment, the invention pertains to the process of the invention further comprising a step of inversion of the configuration at position C3 of the cyclized product of step b). This is based on a two-step oxidation/reduction sequence (Wan et al. Bioorg. Med. Chem. 2004, 12 (13), 3521-3527 and Viton et al. Eur. J. Org. Chem. 2008, (36), 6069-6078). A Dess-Martin oxidation of (R,S)- catechin derivatives leads to the corresponding ketone compounds with 2R absolute stereochemistry without deterioration of optical purity. Subsequent diastereoselective hydride reduction with L-Selectride affords the corresponding (2R, 3R)- epicatechin derivatives while preserving the optical purity. Hence, this step in the production process allows accessing epicatechin conjugate molecules with high optical purity.

In a preferred embodiment of the process of the invention, the trans-cyclization reaction step b) is performed in a two steps sequence, first in the presence of triethylorthoacetate and then followed by a deacetylation reaction at position C3 under reductive conditions. Cyclization using triethyl orthoformate under catalytic pyridinium p-toluenesulfonate conditions proceeds with high stereoselectivity and advantageously allows isolating the target cyclized products in high purity in the form of protected acetate esters. The resulting 3-acyl protected (R,S)-catechin derivatives can then be de-acetylated under mild reductive conditions employing, e.g., DIBAL-H to affording the desired enantiomerically in high yields and as pure *trans-*(R,*S*)-catechin skeletons. In prior art teaching, this reaction sequence is performed in one-pot, the elimination of the intermediate acetate esters being carried out by alkaline methanolysis (Wan et al. Bioorg. Med. Chem. 2004, 12 (13), 3521-3527). This has the disadvantage to result in mixtures of *trans*/*cis* cyclized compounds, which is avoided by the process of present the invention.

For the process of the invention, the protecting group of the compounds of the formula (II) and/or (III) can be selected without limitations from the group consisting of e.g. allyl ether, benzyl ether, tert-butyl ether, tetrahydropyranyl ether, methoxymethyl ether, trimethylsilyl ether tert-butyldimethylsilyl (TBDMS), tert-Butyldiphenylsilyl ether, trimethylsilyl ether, tert-butyldimethylsilyl. Preferably, the protecting group is selected from benzyl and/or TBDMS. This has the advantage of robustness, compatibility with conjugates introduction/deprotection and mild and orthogonal deprotection conditions.

The process of the invention further comprises a reaction step of conjugating the cyclized product of step b) of claim 1 or said product after the inversion of the configuration as of claim 4, with a sulfate donor, a glucuronic acid donor and/or a glucose donor. This reaction step allows generating combination molecules where for example one of the conjugates of the B ring is sulfate while the second conjugate on that same ring is a methyl group. Further combinations can be glucuronidation and methylation in ring B or glycosilation and methylation in ring B. For the above process step, the sulfate donor is preferably selected from neopentylchlorosulfate or trichloroethylchlorosulfate, the glucuronic acid donor is preferably selected from 2,3,4-tri-*O*-acetyl- α,-D-methylglucuronopyranosyl-1-(N-phenyl)-2,2,2-trifluoroacetimidate, 2,3,4-tri-*O*-acetyl- α-D-methylglucopyranosyl-1-(N-4-methoxyphenyl)-2,2,2-trifluoroacetimidate, 2,3,4-tri-*O*-acetyl-α-D-methylglucuronopyranosyl-1-*O*-(2,2,2-trichloroacetimidate) or acetobromo-α-D-glucuronic acid methyl ester, and the glucose donor is preferably selected from 2,3,4,6-tetra-*O*-acetyl- α-D-glucopyranosyl-1-(*N*-phenyl)-2,2,2-trifluoroacetimidate, 2,3,4,6-tetra-*O*-acetyl-α-D-glucuropyranosyl-1-*O*-(2,2,2-trichloroacetimidate) or α-acetobromoglucose.

Another aspect of the invention is the compound of claim 9, wherein in a preferred embodiment R¹ is not equal R² of the formula (I). This pertains for example to a compound wherein the B ring of the compound is conjugated at one of the positions 3' or 4' with methyl while being conjugated at the other corresponding position with either sulfonyl, glucuronyl or glucoside. In a still further embodiment, however, R¹ of the formula (I) is not glucuronyl or glucoside when R² is sulfonyl, or vice versa.

Preferrably, the compounds of the invention are selected from the group consisting of 4'-*O-*methyl-(-)-epicatechin-3'-sulfate; 3'-*O*-methyl-(-)-epi catechin-4'- sulfate; 4'-*O*-methyl-(-)-epicatechin-3'-*O*-β-D-glucuronide; 3'-*O*-methyl-(-)-epicatechin-4'-*O*- β-D-glucuronide; 3'-*O*-methyl-(-)-epicatechin-4'-*O*- β-D-glycoside; 4'-*O*-methyl-(-)-epicatechin-3'-*O*-β-D-glycoside.

A further aspect of the invention is a human or animal food product comprising the compounds of the invention. The compounds of the invention can be used as a food ingredient due to their higher stability, better solubility in water, improved absorption in the gut and their higher inhibitory effect on Maillard reactions than aglycones, and be mixed directly into a variety of different food products. Thereby, food products already naturally comprising flavonoids and/or flavanols can be enriched with the corresponding conjugates to for example provide an improved health beneficial property to a consumer. Alternatively, those compounds can be added to food products naturally not containing such polyphenolic compounds from plants and hence lead to the production of completely novel functional food products. A still further possibility is to use those compounds in nutritional, parenteral solutions as they are used in clinical health care. Thereby, the beneficial effect of the compounds of the invention will be absorbed directly by the body and bypass the intestinal absorption barrier.

In a food product comprising the compound of the invention, the compound is present in a concentration of at least 0.01 mg/g wt%, preferably of at least 0.1 mg/g wt%, more preferably of at least 1 mg/g wt%, even more preferably of 10 mg/g wt% of the total food product, or more.

Those skilled in the art will understand that they can freely combine all features of the present invention disclosed herein. In particular, features described for the process of the invention may be combined with the compound and product of the invention and vice versa. Further, features described for different embodiments of the present invention may be combined.

Further advantages and features of the present invention are apparent from the figures and examples.

### Example 1

### Synthesis of Isomerically Pure Cinnamyl Alcohols

### Example 1a: Synthesis of (E)-2-(benzyloxy)-4-(3-hydroxyprop-1-en-1-yl)phenol.

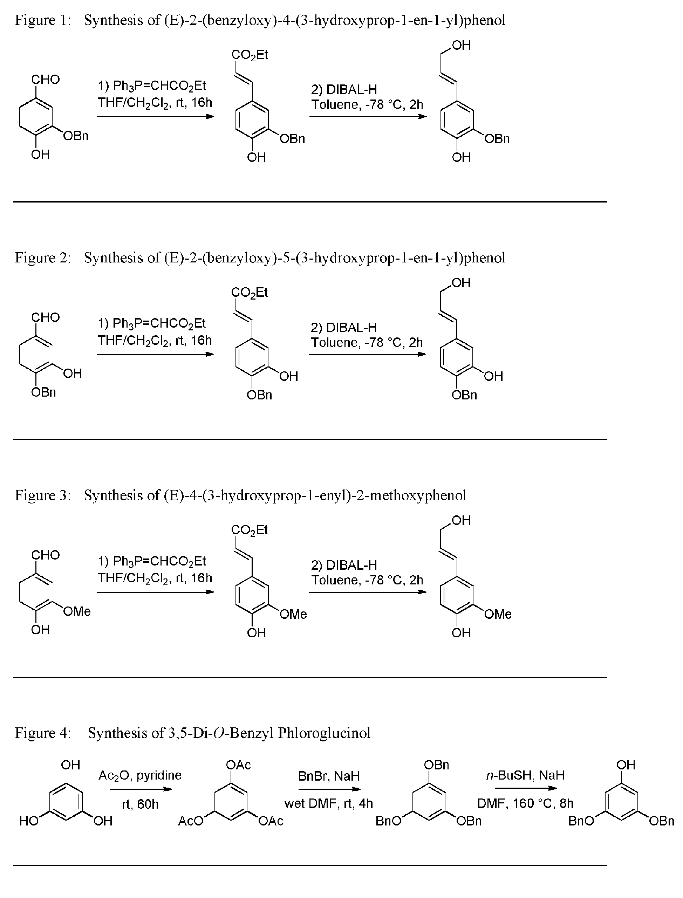

The synthesis of the title compound is illustrated on Figure 1.

### (E)-ethyl 3-(3-benzyloxy-4-hydroxyphenyl)acrylate

The E-selective Wittig olefination reaction was performed inside a flame-dried 100 mL two-necked round-bottomed flask, equipped with a magnetic stirrer bar and capped with a rubber septum, according to an experimental procedure adapted from the works of Georg et al. (J. Org. Chem. 2001, 66 (24), 8211-8214) and Couladouros et al. (Chem. Eur. J. 1998, 4 (1), 33-43). Accordingly, purified (isomerically pure) 3-benzyloxy-4-hydroxybenzaldehyde (1.87 g, 8.19 mmol, 1.0 equiv.) was dissolved in a 1:1 binary mixture of anhydrous tetrahydrofuran (15 mL) and anhydrous methylene chloride (15 mL). The required phosphorus ylide (ethyl (triphenylphosphoranyliden)acetate, 3.1 g, 9.0 mmol, 1.1 equiv.) was then added portionwise as a solid to a vigorously stirred solution of at ambient temperature under a stream of dry argon. The resulting yellow-orange solution was stirred under an argon atmosphere at ambient temperature during overnight. The extent of reaction was followed closely by TLC on silica gel (n-hexane/EtOAc 4:1). Upon completion (≈ 12 h), the crude reaction mixture was poured into water, and the organic residue was extracted with ethyl acetate. The combined organic extracts were washed once with water and once with saturated brine solution, then dried over anhydrous Na2SO4, filtered and concentrated under reduced pressure. The crude product (dark-brown oil, a roughly 98:2 mixture of trans and cis isomers, according to 1H-NMR analysis) was then purified by column chromatography on silica gel employing an elution gradient of ethyl acetate in n-hexane, affording the desired geometrically pure (E)-ethyl 3-(3-benzyloxy-4-hydroxyphenyl)acrylate as a white crystalline solid. Isolated yield 77% (1.89 g, 6.34 mmol). Mp = 126.4-126.8 °C. Rf = 0.32 (silica gel, n-hexane/EtOAc 4: 1). 1H-NMR (400 MHz, CDC13): δ 7.60 (1H, d, Jtrans = 15.9 Hz, olefinic CH=C), 7.41-7.46 (5H, m, benzylic CarH), 7.08-7.11 (2H, m, CarH), 6.94 (1H, d, Jortho = 8.1 Hz, CarH), 6.26 (1H, d, Jtrans = 15.9 Hz, olefinic CH=C), 5.91 (1H, s, hydroxylic OH), 5.13 (2H, s, benzylic CH2), 4.25 (2H, q, J = 7.1 Hz, ethyloxy CH2), 1.33 (3H, t, J = 7.1 Hz, ethyloxy CH3) ppm. 13C-NMR (100 MHz, CDC13): δ 167.4 (carbonyl Cq), 148.3 (Cq), 146.1 (Cq), 144.7 (CH), 136.0 (Cq), 129.0 (CH), 128.8 (CH), 128.1 (CH), 127.2 (Cq), 123.3 (CH), 115.9 (CH), 115.1 (CH), 111.1 (CH), 71.4 (benzylic CH2), 60.5 (ethyloxy CH2), 14.5 (ethyloxy CH3) ppm.

### (E)-2-(benzyloxy)-4-(3-hydroxyprop-1-en-1-yl)phenol

The regioselective 1,2-reduction of (E)-ethyl 3-(3-benzyloxy-4-hydroxyphenyl)acrylate was carried out inside a flame-dried 100 mL two-necked round-bottomed flask, equipped with a magnetic stirrer bar and capped with a rubber septum, according to an experimental procedure adapted from the works of Barron et al. (Eur. J. Org. Chem. 2008, (36), 6069-6078). Accordingly, to a vigorously stirred solution of (E)-ethyl 3-(3-benzyloxy-4-hydroxyphenyl)acrylate (1.87 g, 6.32 mmol, 1.0 equiv.) in anhydrous toluene (37 mL) cooled down to -78 °C (iso-propanol/dry ice bath) was added diisobutylaluminium hydride DIBAL-H (1.0 M solution in toluene, 20.2 mL, 20.2 mmol, 3.2 equiv.) dropwise via syringe under a stream of dry argon (careful, hydrogen gas evolution). The resulting yellow solution was stirred under an argon atmosphere for 2 h at -78 °C, followed by 30 min at ambient temperature (at this stage, the reaction mixture became colourless). Excess hydride was quenched by the addition of excess ethyl acetate, then water and IN HCl aqueous solution were added sequentially, leading to the formation of an organic precipitate. The organic precipitate was extracted with ethyl acetate. The combined organic extracts were washed once with water and once with saturated brine solution, dried over anhydrous Na2SO4 filtered and concentrated in vacuo. The crude (E)-2-(benzyloxy)-4-(3-hydroxyprop-1-en-1-yl)phenol (white amorphous solid, 1.64 g, 6.32 mmol) was recovered in quantitative yield and used directly for the next step, without further purification. Mp = 99.0-101.4 °C. Rf = 0.11 (silica gel, n-hexane/EtOAc 4:1). 1H-NMR (400 MHz, CDC13): δ 7.36-7.42 (5H, m, benzylic CarH), 6.99 (1H, d, Jmeta = 1.6 Hz, CarH), 6.88-6.93 (2H, m, CarH), 6.51 (1H, dt, J1 = 15.8 Hz, J2 = 1.3 Hz, olefinic CH=C), 6.19 (1H, dt, J1 = 15.8 Hz, J2 = 5.9 Hz, olefinic CH=C), 5.75 (1H, s, hydroxylic OH), 5.11 (2H, s, benzylic CH2), 4.28 (2H, d, J = 5.9 Hz, allylic CH2-OH), 1.48 (1H, broad s, hydroxylic OH) ppm. 13C-NMR (100 MHz, CDC13): δ 146.0 (Cq), 145.9 (Cq), 136.3 (Cq), 131.4 (CH), 129.4 (Cq), 128.9 (CH), 128.6 (CH), 128.0 (CH), 126.4 (CH), 120.8 (CH), 114.9 (CH), 110.1 (CH), 71.3 (benzylic CH2), 64.0 (allylic CH2-OH) ppm.

### Example 1b: Synthesis of (E)-2-(benzyloxy)-5-(3-hydroxyprop-1-en-1-yl)phenol.

The synthesis of the title compound is illustrated on Figure 2. It was performed following the procedures described in Example 1a, starting from 4-benzyloxy-3-hydroxybenzaldehyde. Mp = 112.9-113.8 °C. Rf = 0.05 (silica gel, n-hexane/EtOAc 4:1). 1H-NMR (400 MHz, CDC13): δ 7.31-7.39 (5H, m, benzylic CarH), 7.03 (1H, d, Jmeta = 1.9 Hz, CarH), 6.82-6.88 (2H, m, CarH), 6.52 (1H, dt, J1 = 15.8 Hz, J2 = 1.4 Hz, olefinic CH=C), 6.23 (1H, dt, J1 = 15.8 Hz, J2 = 5.9 Hz, olefinic CH=C), 5.64 (1H, s, hydroxylic OH), 5.11 (2H, s, benzylic CH2), 4.29 (2H, td, J1 = 5.8 Hz, J2 = 1.4 Hz, allylic CH2-OH), 1.37 (1H, t, J = 5.8 Hz, hydroxylic OH) ppm. 13C-NMR (100 MHz, CDC13): δ 146.1 (Cq), 145.8 (Cq), 136.4 (Cq), 131.0 (CH), 130.9 (Cq), 128.9 (CH), 128.6 (CH), 128.0 (CH), 127.1 (CH), 119.1 (CH), 112.4 (CH), 112.3 (CH), 71.4 (benzylic CH2), 64.0 (allylic CH2-OH) ppm. LC/ESI-TOF-MS (-) (m/z): calc. for C16H15O3 [M-H]- 255.1021, obs. 255.1016; calc. for C9H8O3 [M-Bn-H]-164.0473, obs. 164.0472.

### Example 1c: Synthesis of (E)-4-(3-hydroxyprop-1-enyl)-2-methoxyphenol.

The synthesis of the title compound is illustrated on Figure 3. It was performed following the procedures described in Example 1a, starting from vanillin.

Rf = 0.11 (silica gel, n-hexane/EtOAc 4:1). 1H-NMR (400 MHz, CDC13): δ 6.91 (1H, d, Jmeta = 1.5 Hz, CarH), 6.87 (2H, AB q, Jortho = 7.6 Hz, CarH), 6.52 (1H, dt, Jltrans = 15.8 Hz, J2 = 1.4 Hz, olefinic CH=C), 6.21 (1H, dt, Jltrans = 15.8 Hz, J2 = 6.0 Hz, olefinic CH=C), 5.75 (1H, s, phenolic OH), 4.29 (1H, broad s, allylic CH2), 3.89 (3H, s, methyloxy CH3), 1.66 (1H, broad s, hydroxylic OH) ppm. 13C-NMR (100 MHz, CDC13): δ 146.8 (Cq), 145.7 (Cq), 131.5 (CH), 129.4 (Cq), 126.3 (CH), 120.4 (CH), 114.6 (CH), 108.5 (CH), 64.0 (methyloxy CH3), 56.0 (allylic CH2) ppm.

### Example 2

### Synthesis of 3,5-Di-O-Benzyl Phloroglucinol

The synthesis of the title compound is illustrated on Figure 4.

### Benzene-1,3,5-triyl triacetate

The per-O-acetylation reaction of phloroglucinol was carried out inside a flame-dried 250 mL two-necked round-bottomed flask, equipped with a magnetic stirrer bar, an addition funnel and capped with a rubber septum, according to an experimental procedure adapted from the works of Murakami et al. (Synth. Commun. 1996, 26 (3), 531-534). Accordingly, phloroglucinol (29.71 g, 236 mmol, 1.0 equiv.) was suspended in acetic anhydride Ac20 (90 mL, 944 mmol, 4.0 equiv.) and the mixture was cooled down to 0 °C (ice/water bath). Freshly distilled pyridine (90 mL, 1.11 mol, 4.7 equiv.) was added dropwise via addition funnel under a stream of dry argon, the solid residue was dissolved and the resulting colourless solution was stirred under an argon atmosphere at ambient temperature for 60 h. Upon completion (monitoring by TLC on silica gel, n-hexane/EtOAc 2:1), the crude reaction mixture was poured into ice-water and the obtained organic precipitate was filtered carefully under vacuum. The white precipitate was further rinsed abundantly with water and finally dried inside a vacuum oven at 60 °C for at least one day. The chemical purity of the recovered crude benzene-1,3,5-triyl triacetate was assessed as sufficient by 1H-NMR spectral analysis. The crude product (white amorphous solid, 49.63 g, 197.2 mmol) was recovered in 83% crude yield and used directly for the next step, without further purification. An analytical sample of benzene-1,3,5-triyl triacetate was obtained after a single recrystallization from ethanol. White needle-shaped crystals. Mp = 105.4-105.6 °C. Rf = 0.50 (silica gel, n-hexane/EtOAc 2: 1). 1H-NMR (400 MHz, CDC13): δ 6.84 (3H, s, CarH), 2.27 (9H, s, acetyl CH3) ppm. 13C-NMR (100 MHz, CDC13): δ 168.6 (carbonyl Cq), 151.2 (Cq), 112.8 (CH), 21.2 (acetyl CH3) ppm.

### 1,3,5-tris(benzyloxy)benzene

The per-O-benzylation reaction of benzene-1,3,5-triyl triacetate was carried out inside a flame-dried 500 mL three-necked round-bottomed flask, equipped with a magnetic stirrer bar and capped with a rubber septum, according to an experimental procedure adapted from the works of Murakami et al. (Synth. Commun. 1996, 26 (3), 531-534). Accordingly, benzene-1,3,5-triyl triacetate (10 g, 39.6 mmol, 1.0 equiv.) was dissolved in anhydrous DMF (200 mL) and the obtained colourless solution was cooled down to 0 °C with help of an ice/water cooling bath. Benzyl bromide (16.9 mL, 142.6 mmol, 3.6 equiv.) was added in one portion via syringe, followed by sodium hydride (60% dispersion in mineral oils, 11.4 g, 285.1 mmol, 7.2 equiv.), under a stream of dry argon. After stirring for 15 min at 0 °C under an argon atmosphere (careful, hydrogen gas evolution), distilled water was added portionwise via syringe (in 10 portions of 200 µL each) while maintaining the temperature close to 0 °C. After complete addition of water, the reaction mixture was left stirring for 4 h at ambient temperature. Upon completion (monitoring by TLC on silica gel, n-hexane/EtOAc 4:1), the reaction medium was poured into ice-cooled IN HCl aqueous solution and the separated organic residue was extracted several times with methylene chloride. The combined organic layers were washed once with water and once with saturated brine solution, dried over anhydrous Na2SO4, filtered and concentrated in vacuo. The chemical purity of the recovered crude 1,3,5-tris(benzyloxy)benzene was assessed as sufficient by 1H-NMR spectral analysis. The crude product (white amorphous solid, 11.43 g, 28.8 mmol) was recovered in 73% crude yield and used directly for the next step, without further purification. An analytical sample of 1,3,5-tris(benzyloxy)benzene was obtained after a single recrystallization from ethanol. White needle-shaped crystals. Mp = 91.5-91.9 °C. Rf = 0.75 (silica gel, n-hexane/EtOAc 4: 1). 1H-NMR (400 MHz, CDC13): δ 7.34-7.45 (15H, m, benzylic CarH), 6.29 (3H, s, CarH), 5.02 (6H, s, benzylic CH2) ppm. 13C-NMR (100 MHz, CDC13): δ 160.8 (Cq), 137.0 (Cq), 128.7 (CH), 128.1 (CH), 127.7 (CH), 95.1 (CH), 70.3 (benzylic CH2) ppm.

### 3,5-Di-O-Benzyl Phloroglucinol

The chemoselective mono-debenzylation reaction of 1,3,5-tris(benzyloxy)benzene was carried out inside a flame-dried 250 mL two-necked round-bottomed flask, equipped with a magnetic stirrer bar, a water-jacketed condenser and capped with a rubber septum, according to an experimental procedure adapted from the works of Lau et al. (Tetrahedron 1998, 54 (45), 13813-13824). Accordingly, a 60% suspension of sodium hydride NaH in mineral oils (1.21 g, 28.75 mmol, 1.9 equiv.) was loaded inside the flask and washed three times with dry n-hexane under a stream of dry argon. Following the complete evaporation of traces of n-hexane with help of an oil pump, anhydrous DMF (30 mL) was added and the resulting white suspension was cooled down to 0 °C (ice/water bath) under an argon atmosphere. Under vigorous stirring of the reaction medium, freshly purchased n-butanethiol (n-BuSH, 3.11 mL, 28.75 mmol, 1.9 equiv.) was added dropwise via syringe over a period of 10 min (careful, hydrogen gas evolution). The white suspension was then stirred under an argon atmosphere for 1.5 h at 0 °C followed by 20 min at ambient temperature, until a transparent yellow solution eventually formed. A solution of 1,3,5-tris(benzyloxy)benzene (6.0 g, 15.13 mmol, 1.0 equiv.) in anhydrous DMF (30 mL) was then added in one portion via syringe and the reaction mixture was refluxed under argon (≈ 160 °C) during 6-8 h (monitoring by TLC on silica gel, n-hexane/EtOAc 4:1). Upon completion, the obtained dark-orange solution was diluted with ethyl acetate and excess hydride was quenched by the addition of IN HCl aqueous solution. After separation of the two layers, the aqueous layer was extracted with ethyl acetate. The combined organic layers were washed abundantly with water (removal of DMF), dried over anhydrous Na2SO4, filtered and concentrated under reduced pressure. The crude product (orange oil) was purified by column chromatography on silica gel employing an elution gradient of ethyl acetate in n-hexane, affording the desired pure 3,5-bis(benzyloxy)phenol as a white crystalline solid. Isolated yield 84% (3.87 g, 12.61 mmol). Mp = 89.4-90.4 °C. Rf = 0.30 (silica gel, n-hexane/EtOAc 4:1). 1H-NMR (400 MHz, CDC13): δ 7.31-7.42 (10H, m, benzylic CarH), 6.24 (1H, t, Jmeta = 2.1 Hz, CarH), 6.11 (2H, d, Jmeta = 2.1 Hz, CarH), 5.00 (4H, s, benzylic CH2), 4.75 (1H, s, hydroxylic OH) ppm. 13C-NMR (100 MHz, CDC13): δ 160.8 (Cq), 157.2 (Cq), 136.8 (Cq), 128.6 (CH), 128.0 (CH), 127.5 (CH), 95.4 (CH), 95.0 (CH), 70.1 (benzylic CH2) ppm.

### Example 3

### Friedel-Crafts Coupling Between 3,5-Di-O-Benzyl Phloroglucinol and Cinnamyl Alcohol

### Example 3a: Synthesis of (E)-3,5-bis(benzyloxy)-2-(3-(3-(benzyloxy)-4-hydroxyphenyl)allyl)phenol.

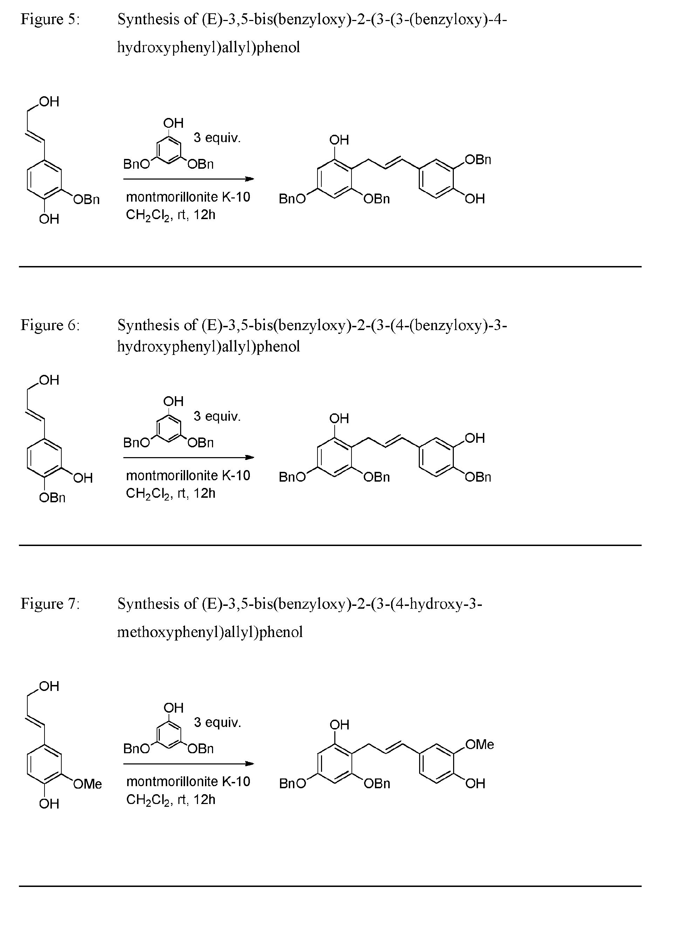

The synthesis of the title compound is illustrated on Figure 5.

Reagents: 3,5-bis(benzyloxy)phenol (5.51 g, 18.0 mmol, 3.0 equiv.) and Montmorillonite K-10 clay (5.51 g, 0.3 g/mmol) in anhydrous methylene chloride (245 mL), (E)-2-(benzyloxy)-4-(3-hydroxyprop-1-en-1-yl)phenol (1.54 g, 6.0 mmol, 1.0 equiv.) in anhydrous methylene chloride (125 mL). Slow addition at ambient temperature over a period of 1.5 h. Leave stirring the resulting purple suspension for 12 h at ambient temperature under an argon atmosphere. The crude product (brown-orange solid residue) was purified by column chromatography on silica gel employing an elution gradient of ethyl acetate in n-hexane, affording the desired pure (E)-3,5-bis(benzyloxy)-2-(3-(3-(benzyloxy)-4-hydroxyphenyl)allyl)phenol as a white amorphous solid. Isolated yield 62% (2.02 g, 3.71 mmol). Mp = 126.2-130.6 °C (decomposition). Rf = 0.23 (silica gel, n-hexane/EtOAc 4: 1). 1H-NMR (400 MHz, CDC13): δ 7.30-7.42 (15H, m, benzylic CarH), 6.94 (1H, broad s, CarH), 6.85 (2H, broad s, A2 spin-system, CarH), 6.39 (1H, broad d, Jtrans = 15.8 Hz, olefinic CH=C), 6.29 (1H, d, Jmeta = 2.3 Hz, CarH), 6.18 (1H, d, Jmeta = 2.3 Hz, CarH), 6.13 (1H, dt, Jltrans = 15.8 Hz, J2 = 6.4 Hz, olefinic CH=C), 5.59 (1H, broad s, phenolic OH), 5.07 (2H, s, benzylic CH2), 5.06 (1H, broad s, phenolic OH), 5.03 (2H, s, benzylic CH2), 5.01 (2H, s, benzylic CH2), 3.57 (2H, dd, J1 = 6.4 Hz, J2allylic = 1.4 Hz, allylic CH2) ppm. 13C-NMR (100 MHz, CDC13): δ 159.0 (Cq), 158.0 (Cq), 156.0 (Cq), 146.0 (Cq), 145.5 (Cq), 137.3 (Cq), 137.0 (Cq), 136.4 (Cq), 130.6 (CH), 130.1 (Cq), 128.9 (CH), 128.8 (CH), 128.7 (CH), 128.6 (CH), 128.2 (CH), 128.1 (CH), 128.0 (CH), 127.7 (CH), 127.4 (CH), 126.2 (CH), 120.4 (CH), 114.7 (CH), 109.8 (CH), 107.1 (Cq), 95.3 (CH), 93.9 (CH), 71.3 (benzylic CH2), 70.5 (benzylic CH2), 70.3 (benzylic CH2), 26.5 (allylic CH2) ppm. 1H-NMR (400 MHz, DMSO-d6): δ 9.34 (1H, s, phenolic OH), 8.97 (1H, s, phenolic OH), 7.29-7.48 (15H, m, benzylic CarH), 6.93 (1H, d, Jmeta = 1.7 Hz, CarH), 6.71 (1H, d, Jortho = 8.2 Hz, CarH), 6.67 (1H, dd, Jlortho = 8.2 Hz, J2meta = 1.7 Hz, CarH), 6.26 (1H, d, Jmeta = 2.3 Hz, CarH), 6.15 (1H, d, Jtrans = 15.8 Hz, olefinic CH=C), 6.14 (1H, d, Jmeta = 2.3 Hz, CarH), 6.04 (1H, dt, Jltrans = 15.8 Hz, J2 = 6.2 Hz, olefinic CH=C), 5.08 (2H, s, benzylic CH2), 5.07 (2H, s, benzylic CH2), 5.00 (2H, s, benzylic CH2), 3.34 (2H, d, J = 6.2 Hz, allylic CH2) ppm. 13C-NMR (100 MHz, DMSO-d6): δ 157.7 (Cq), 157.6 (Cq), 156.2 (Cq), 146.6 (Cq), 146.1 (Cq), 137.5 (Cq), 137.4 (Cq), 137.2 (Cq), 129.1 (CH), 128.9 (Cq), 128.4 (CH), 128.3 (CH), 128.2 (CH), 127.7 (CH), 127.6 (2 x CH), 127.5 (2 x CH), 127.2 (CH), 126.0 (CH), 119.2 (CH), 115.8 (CH), 111.2 (CH), 107.2 (Cq), 94.8 (CH), 91.9 (CH), 69.8 (benzylic CH2), 69.2 (benzylic CH2), 69.1 (benzylic CH2), 25.9 (allylic CH2) ppm. LC/ESI-TOF-MS (-) (m/z): calc. for C36H31O5 [M-H]- 543.2171, obs. 543.2084; calc. for C72H63O10 [2M-H]-1087.4421, obs. 1087.4420.

### Example 3b: Synthesis of (E)-3,5-bis(benzyloxy)-2-(3-(4-(benzyloxy)-3-hydroxyphenyl)allyl)phenol.

The synthesis of the title compound is illustrated on Figure 6. It was performed following the procedures described in Example 3a, starting from (E)-2-(benzyloxy)-5-(3-hydroxyprop-1-en-1-yl)phenol.

Mp = 144.9-146.3 °C. Rf = 0.19 (silica gel, n-hexane/EtOAc 4:1). 1H-NMR (400 MHz, CDC13): δ 7.29-7.41 (15H, m, benzylic CarH), 6.96 (1H, d, Jmeta = 2.0 Hz, CarH), 6.82 (1H, d, Jortho = 8.4 Hz, CarH), 6.75 (1H, dd, Jlortho = 8.4 Hz, J2meta = 2.0 Hz, CarH), 6.37 (1H, broad d, Jtrans = 15.8 Hz, olefinic CH=C), 6.28 (1H, d, Jmeta = 2.3 Hz, CarH), 6.17 (1H, d, Jmeta = 2.3 Hz, CarH), 6.17 (1H, dt, Jltrans = 15.8 Hz, J2 = 6.4 Hz, olefinic CH=C), 5.58 (1H, broad s, phenolic OH), 5.09 (2H, s, benzylic CH2), 5.06 (1H, broad s, phenolic OH), 5.03 (2H, s, benzylic CH2), 5.00 (2H, s, benzylic CH2), 3.56 (2H, dd, J1 = 6.4 Hz, J2allylic = 1.4 Hz, allylic CH2) ppm. 13C-NMR (100 MHz, CDC13): δ 159.0 (Cq), 158.0 (Cq), 155.9 (Cq), 146.1 (Cq), 145.3 (Cq), 137.2 (Cq), 137.1 (Cq), 136.5 (Cq), 131.6 (Cq), 130.2 (CH), 128.9 (CH), 128.8 (CH), 128.7 (CH), 128.5 (CH), 128.1 (CH), 128.0 (CH), 127.9 (CH), 127.7 (CH), 127.5 (CH), 127.0 (CH), 118.6 (CH), 112.3 (CH), 112.2 (CH), 107.1 (Cq), 95.3 (CH), 93.9 (CH), 71.4 (benzylic CH2), 70.6 (benzylic CH2), 70.3 (benzylic CH2), 26.5 (allylic CH2) ppm. 1H-NMR (400 MHz, DMSO-d6): δ 9.36 (1H, s, phenolic OH), 8.96 (1H, s, phenolic OH), 7.31-7.47 (15H, m, benzylic CarH), 6.87 (1H, d, Jortho = 8.4 Hz, CarH), 6.78 (1H, d, Jmeta = 2.1 Hz, CarH), 6.61 (1H, dd, Jlortho = 8.4 Hz, J2meta = 2.1 Hz, CarH), 6.26 (1H, d, Jmeta = 2.3 Hz, CarH), 6.15 (1H, d, Jmeta = 2.3 Hz, CarH), 6.14 (1H, broad d, Jtrans = 15.8 Hz, olefinic CH=C), 6.03 (1H, dt, Jltrans = 15.8 Hz, J2 = 6.3 Hz, olefinic CH=C), 5.08 (4H, s, 2 x benzylic CH2), 5.01 (2H, s, benzylic CH2), 3.35 (2H, broad d, J = 6.3 Hz, allylic CH2) ppm. 13C-NMR (100 MHz, DMSO-d6): δ 157.8 (Cq), 157.6 (Cq), 156.2 (Cq), 146.9 (Cq), 145.7 (Cq), 137.5 (Cq), 137.4 (Cq), 137.2 (Cq), 131.0 (Cq), 128.6 (CH), 128.5 (CH), 128.4 (CH), 128.3 (CH), 128.2 (CH), 127.7 (CH), 127.6 (2 x CH), 127.4 (CH), 127.2 (CH), 126.8 (CH), 117.1 (CH), 114.3 (CH), 112.6 (CH), 107.1 (Cq), 94.8 (CH), 91.9 (CH), 69.9 (benzylic CH2), 69.2 (benzylic CH2), 69.1 (benzylic CH2), 25.9 (allylic CH2) ppm.

### Example 3c: Synthesis of (E)-3,5-bis(benzyloxy)-2-(3-(4-hydroxy-3-methoxyphenyl)allyl)phenol.

The synthesis of the title compound is illustrated on Figure 7. It was performed following the procedures described in Example 3a, starting from (E)-4-(3-hydroxyprop-1-enyl)-2-methoxyphenol.

Rf = 0.22 (silica gel, n-hexane/EtOAc 7:3). 1H-NMR (400 MHz, CDC13): δ 7.29-7.43 (10H, m, benzylic CarH), 6.81-6.84 (3H, m, CarH), 6.41 (1H, dt, Jltrans = 15.8 Hz, J2allylic = 1.5 Hz, olefinic CH=C), 6.29 (1H, d, Jmeta = 2.3 Hz, CarH), 6.18 (1H, d, Jmeta = 2.3 Hz, CarH), 6.15 (1H, dt, Jltrans = 15.8 Hz, J2 = 6.4 Hz, olefinic CH=C), 5.56 (1H, broad s, phenolic OH), 5.11 (1H, broad s, phenolic OH), 5.04 (2H, s, benzylic CH2), 5.01 (2H, s, benzylic CH2), 3.87 (3H, s, methyloxy CH3), 3.58 (2H, dd, J1 = 6.4 Hz, J2allylic = 1.5 Hz, allylic CH2) ppm. 13C-NMR (100 MHz, CDC13): δ 159.0 (Cq), 158.0 (Cq), 156.0 (Cq), 146.7 (Cq), 145.2 (Cq), 137.3 (Cq), 137.0 (Cq), 130.6 (CH), 130.0 (Cq), 128.8 (CH), 128.7 (CH), 128.2 (CH), 128.0 (CH), 127.7 (CH), 127.4 (CH), 126.1 (CH), 120.0 (CH), 114.4 (CH), 108.2 (CH), 107.2 (Cq), 95.3 (CH), 93.9 (CH), 70.5 (benzylic CH2), 70.3 (benzylic CH2), 56.0 (methyloxy CH3), 26.5 (allylic CH2) ppm.

### Example 4

### Sharpless Asymmetric Dihydroxylation and Elaboration of Enantiomerically Pure Tetraols

### Example 4a: Synthesis of (1S,2S)-1-(3-(benzyloxy)-4-((tert-butyldimethylsilyl)oxy)phenyl)-3-(2,4-bis(benzyloxy)-6-((tert-butyldimethylsilyl)oxy)phenyl)propane-1,2-diol.

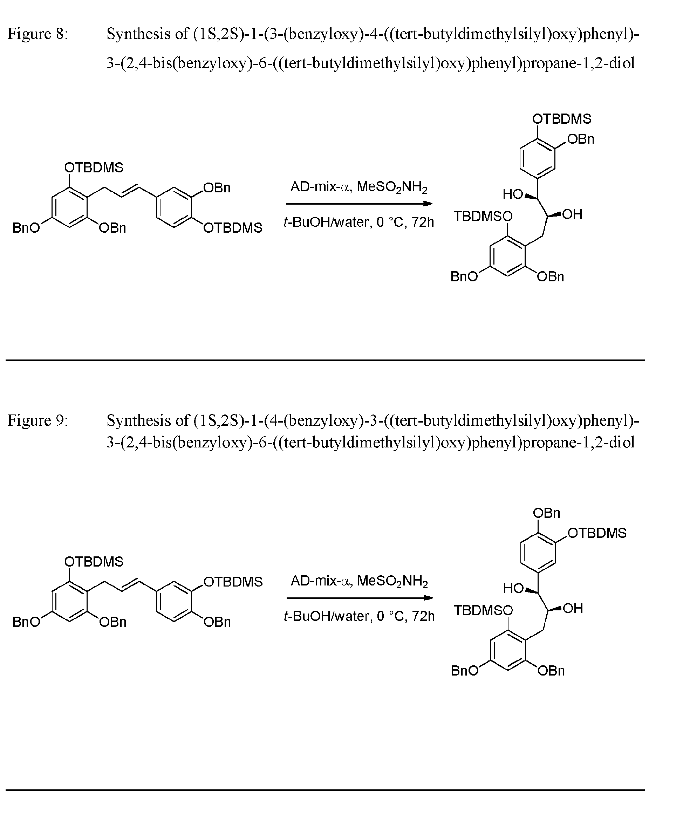

The synthesis of the title compound is illustrated on Figure 8.

Reagents: the commercially available Sharpless asymmetric dihydroxylation reagent (AD-mix-a, 22.33 g, 7.0 g/mmol) and methanesulfonamide (1.52 g, 15.95 mmol, 5.0 equiv.) in a binary 1:1 solvent mixture of tert-butanol (35 mL) and water (35 mL), (E)-(2-(benzyloxy)-4-(3-(2,4-bis(benzyloxy)-6-((tert-butyldimethylsilyl)oxy) phenyl)prop-1-en-1-yl)phenoxy)(tert-butyl)dimethylsilane (2.47 g, 3.19 mmol, 1.0 equiv.) in anhydrous methylene chloride (32 mL). The orange coloured biphasic mixture was stirred vigorously at 0 °C under an argon atmosphere for 72 h. The crude product (orange-brown oil) was purified by column chromatography on silica gel employing an elution gradient of ethyl acetate in n-hexane, affording the desired pure (1S,2S)-1-(3-(benzyloxy)-4-((tert-butyldimethylsilyl)oxy)phenyl)-3-(2,4-bis(benzyloxy)-6-((tert-butyldimethylsilyl)oxy)phenyl)propane-1,2-diol as a very viscous colourless oil. Isolated yield 92% (2.37 g, 2.94 mmol). Rf = 0.34 (silica gel, n-hexane/EtOAc 4:1). 1H-NMR (400 MHz, CDC13): δ 7.27-7.36 (15H, m, benzylic CarH), 6.96 (1H, d, Jmeta = 1.5 Hz, CarH), 6.75-6.80 (2H, m, AB spin-system, CarH), 6.30 (1H, d, Jmeta = 2.3 Hz, CarH), 6.11 (1H, d, Jmeta = 2.3 Hz, CarH), 4.99 (2H, s, benzylic CH2), 4.98 (2H, s, benzylic CH2), 4.93-4.97 (2H, m, AB spin-system, benzylic CH2), 4.39 (1H, dd, J1 = 5.2 Hz, J2 = 3.3 Hz, Car-CH-OH), 3.85 (1H, dq, J1 = 7.9 Hz, J2 = 6.1 Hz, CH2-CH-OH), 3.17 (1H, d, J = 3.3 Hz, hydroxylic OH), 2.78-2.81 (2H, m, diastereotopic CH2, ABX spin-system), 2.54 (1H, d, J = 5.5 Hz, hydroxylic OH), 0.96 (9H, s, Si-C(CH3)3), 0.95 (9H, s, Si-C(CH3)3), 0.17 (3H, s, Si-CH3), 0.15 (3H, s, Si-CH3), 0.07 (6H, s, 2 x Si-CH3) ppm. 1H-NMR (400 MHz, DMSO-d6): δ 7.26-7.43 (15H, m, benzylic CarH), 6.97 (1H, broad s, CarH), 6.71 (2H, broad s, A2 spin-system, CarH), 6.35 (1H, d, Jmeta = 2.3 Hz, CarH), 5.97 (1H, d, Jmeta = 2.3 Hz, CarH), 5.02 (4H, s, 2 x benzylic CH2), 5.00 (1H, d, J = 4.7 Hz, hydroxylic OH), 4.94 (2H, s, benzylic CH2), 4.25 (1H, t, J = 4.9 Hz, Car-CH-OH), 3.92 (1H, d, J = 5.5 Hz, hydroxylic OH), 3.83 (1H, dq, J1 = 9.6 Hz, J2 = 5.0 Hz, CH2-CH-OH), 2.65 (1H, dd, J1 = 13.2 Hz, J2 = 9.6 Hz, diastereotopic CH2, A part of AB spin-system), 2.47 (1H, dd, J1 = 13.2 Hz, J2 = 4.3 Hz, diastereotopic CH2, B part of AB spin-system), 0.91 (9H, s, Si-C(CH3)3), 0.90 (9H, s, SiC(CH3)3), 0.10 (3H, s, Si-CH3), 0.09 (3H, s, Si-CH3), 0.03 (6H, s, 2 x Si-CH3) ppm. 13C-NMR (100 MHz, DMSO-d6): δ 158.2 (Cq), 157.2 (Cq), 154.7 (Cq), 149.0 (Cq), 143.1 (Cq), 137.4 (Cq), 137.2 (Cq), 137.1 (Cq), 136.9 (Cq), 128.4 (CH), 128.3 (CH), 128.2 (CH), 127.8 (CH), 127.7 (CH), 127.5 (CH), 127.4 (CH), 127.3 (CH), 127.0 (CH), 119.7 (CH), 119.3 (CH), 112.6 (Cq), 111.0 (CH), 98.1 (CH), 94.1 (CH), 76.3 (CH-OH), 73.6 (CH-OH), 69.9 (benzylic CH2), 69.3 (benzylic CH2), 69.2 (benzylic CH2), 25.8 (allylic CH2), 25.7 (Si-C(CH3)3), 25.5 (Si-C(CH3)3), 18.0 (Si-C(CH3)3), 17.9 (Si-C(CH3)3), -4.3 (Si-CH3), -4.4 (Si-CH3), -4.7 (Si-CH3), -4.8 (Si-CH3) ppm.

### Example 4b: Synthesis of (1S,2S)-1-(4-(benzyloxy)-3-((tert-butyldimethylsilyl)oxy)phenyl)-3-(2,4-bis(benzyloxy)-6-((tert-butyldimethylsilyl)oxy)phenyl)propane-1,2-diol.

The synthesis of the title compound is illustrated on Figure 9. It was performed following the procedures described in Example 4a, starting from (E)-(2-(benzyloxy)-5-(3-(2,4-bis(benzyloxy)-6-((tert-butyldimethylsilyl)oxy) phenyl)prop-1-en-1-yl)phenoxy)(tert-butyl)dimethylsilane.

Rf = 0.31 (silica gel, n-hexane/EtOAc 4:1). 1H-NMR (400 MHz, CDC13): δ 7.30-7.41 (15H, m, benzylic CarH), 6.84-6.87 (2H, m, AB spin-system, CarH), 6.80 (1H, d, Jortho = 8.8 Hz, CarH), 6.29 (1H, d, Jmeta = 2.3 Hz, CarH), 6.10 (1H, d, Jmeta = 2.3 Hz, CarH), 5.00 (2H, s, benzylic CH2), 4.99 (4H, s, 2 x benzylic CH2), 4.36 (1H, dd, J1 = 5.0 Hz, J2 = 3.4 Hz, Car-CH-OH), 3.84 (1H, dq, J1 = 8.2 Hz, J2 = 5.5 Hz, CH2-CH-OH), 3.11 (1H, d, J = 3.4 Hz, hydroxylic OH), 2.77-2.83 (2H, m, diastereotopic CH2, ABX spin-system), 2.50 (1H, d, J = 5.5 Hz, hydroxylic OH), 0.95 (9H, s, Si-C(CH3)3), 0.94 (9H, s, Si-C(CH3)3), 0.16 (3H, s, Si-CH3), 0.15 (3H, s, Si-CH3), 0.08 (6H, s, 2 x Si-CH3) ppm. 1H-NMR (400 MHz, DMSO-d6): δ 7.28-7.45 (15H, m, benzylic CarH), 6.93 (1H, d, Jortho = 8.9 Hz, CarH), 6.76-6.78 (2H, m, CarH), 6.34 (1H, d, Jmeta = 2.3 Hz, CarH), 5.97 (1H, d, Jmeta = 2.3 Hz, CarH), 5.02 (6H, s, 3 x benzylic CH2), 4.97 (1H, d, J = 4.6 Hz, hydroxylic OH), 4.22 (1H, t, J = 4.7 Hz, Car-CH-OH), 3.87 (1H, d, J = 5.7 Hz, hydroxylic OH), 3.79 (1H, dq, J1 = 9.2 Hz, J2 = 4.6 Hz, CH2-CH-OH), 2.69 (1H, dd, J1 = 13.6 Hz, J2 = 4.2 Hz, diastereotopic CH2, A part of AB spin-system), 2.47 (1H, dd, J1 = 13.6 Hz, J2 = 9.2 Hz, diastereotopic CH2, B part of AB spin-system), 0.91 (9H, s, Si-C(CH3)3), 0.90 (9H, s, Si-C(CH3)3), 0.10 (3H, s, Si-CH3), 0.09 (3H, s, Si-CH3), 0.03 (3H, s, Si-CH3), 0.02 (3H, s, Si-CH3) ppm. 13C-NMR (100 MHz, DMSO-d6): δ 158.1 (Cq), 157.2 (Cq), 154.7 (Cq), 148.4 (Cq), 143.6 (Cq), 137.4 (Cq), 137.2 (Cq), 137.1 (Cq), 136.3 (Cq), 128.4 (CH), 128.3 (CH), 128.2 (CH), 127.8 (CH), 127.7 (CH), 127.6 (CH), 127.5 (CH), 127.4 (CH), 126.9 (CH), 119.9 (CH), 119.4 (CH), 113.1 (CH), 111.0 (Cq), 98.1 (CH), 94.1 (CH), 76.0 (CH-O), 73.7 (CH-O), 70.0 (benzylic CH2), 69.3 (benzylic CH2), 69.2 (benzylic CH2), 27.3 (allylic CH2), 25.7 (Si-C(CH3)3), 25.5 (Si-C(CH3)3), 18.0 (SiC(CH3)3), 17.9 (Si-C(CH3)3), -4.3 (Si-CH3), -4.4 (Si-CH3), -4.7 (Si-CH3), -4.8 (Si-CH3) ppm.

### Example 4c: Synthesis of (1S,2S)-3-(2,4-bis(benzyloxy)-6-((tert-butyldimethylsilyl)oxy)phenyl)-1-(4-((tert-butyldimethylsilyl)oxy)-3-methoxyphenyl)propane-1,2-diol.

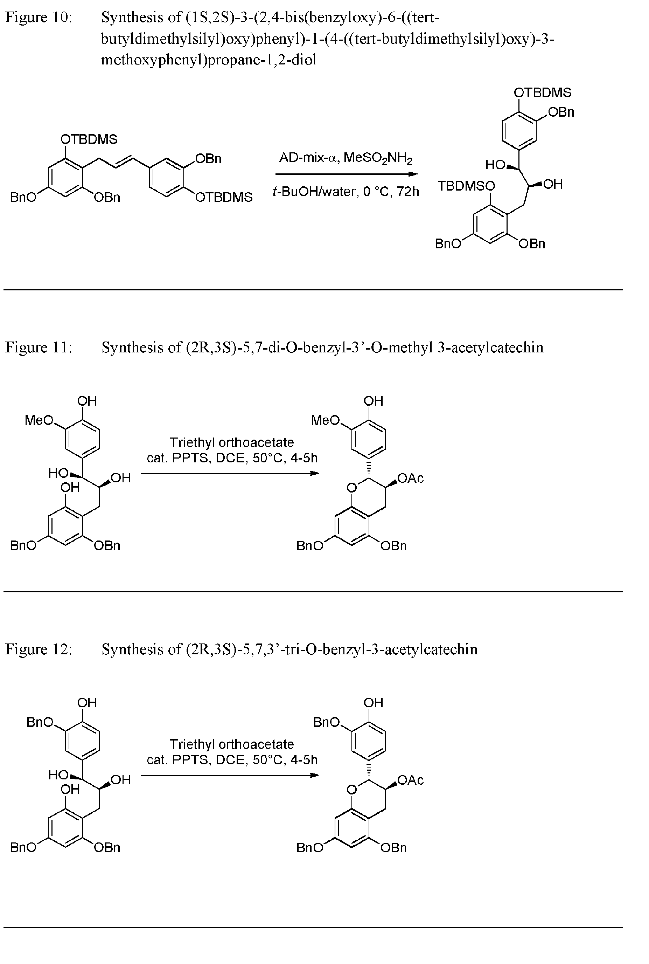

The synthesis of the title compound is illustrated on Figure10. It was performed following the procedures described in Example 4a, starting from (E)-(3,5-bis(benzyloxy)-2-(3-(4-((tert-butyldimethylsilyl)oxy)-3-methoxyphenyl)allyl)phenoxy)(tert-butyl)dimethylsilane. Rf = 0.29 (silica gel, n-hexane/EtOAc 4:1). 1H-NMR (400 MHz, CDC13): δ 7.30-7.39 (10H, m, benzylic CarH), 6.87 (1H, s, CarH), 6.74 (2H, A2 s, CarH), 6.29 (1H, d, Jmeta = 2.3 Hz, CarH), 6.10 (1H, d, Jmeta = 2.3 Hz, CarH), 4.99 (4H, s, 2 x benzylic CH2), 4.40 (1H, dd, J1 = 5.1 Hz, J2 = 3.4 Hz, Car-CH-OH), 3.83-3.91 (1H, m, CH2-CH-OH), 3.73 (3H, s, methyloxy CH3), 3.18 (1H, d, J = 3.3 Hz, hydroxylic OH), 2.76-2.86 (2H, m, diastereotopic CH2, ABX spin-system), 2.53 (1H, d, J = 5.4 Hz, hydroxylic OH), 0.99 (9H, s, Si-C(CH3)3), 0.95 (9H, s, Si-C(CH3)3), 0.16 (3H, s, Si-CH3), 0.15 (3H, s, Si-CH3), 0.13 (3H, s, Si-CH3), 0.12 (3H, s, Si-CH3) ppm. 13C-NMR (100 MHz, CDC13): δ 158.55 (Cq), 158.51 (Cq), 155.4 (Cq), 150.9 (Cq), 144.6 (Cq), 137.0 (Cq), 136.7 (Cq), 134.6 (Cq), 128.9 (CH), 128.8 (CH), 128.2 (CH), 128.1 (CH), 127.5 (CH), 127.3 (CH), 120.7 (CH), 119.3 (CH), 110.8 (CH), 110.1 (Cq), 99.0 (CH), 94.4 (CH), 77.1 (CH-OH), 75.7 (CH-OH), 70.6 (benzylic CH2), 70.4 (benzylic CH2), 55.5 (methyloxy CH3), 27.8 (allylic CH2), 26.0 (Si-C(CH3)3), 25.9 (Si-C(CH3)3), 18.6 (SiC(CH3)3), 18.4 (Si-C(CH3)3), -3.9 (Si-CH3), -4.1 (Si-CH3), -4.4 (Si-CH3), -4.5 (Si-CH3) ppm.

### Example 5

### Cyclization to the Catechin Skeleton

### Example 5a: Synthesis of (2R,3S)-5,7-di-O-benzyl-3'-O-methyl 3-acetylcatechin.

The synthesis of the title compound is illustrated on Figure 11.

The cyclization reaction of (1S,2S)-3-(2,4-bis(benzyloxy)-6-hydroxyphenyl)-1-(4-hydroxy-3-methoxyphenyl)propane-1,2-diol was carried out under essentially neutral reaction conditions, inside a flame-dried 50 mL two-necked round-bottomed flask, equipped with a magnetic stirrer bar and capped with a rubber septum, according to an experimental procedure adapted from the works of Chan et al. (Bioorg. Med. Chem. 2004, 12 (13), 3521-3527) and Barron et al. (Eur. J. Org. Chem. 2008, (36), 6069-6078). Accordingly, to a well-stirred suspension of (1S,2S)-3-(2,4-bis(benzyloxy)-6-hydroxyphenyl)-1-(4-hydroxy-3-methoxyphenyl)propane-1,2-diol (1.85 g, 3.42 mmol, 1.0 equiv.) in anhydrous 1,2-dichloroethane (DCE, 47 mL) was added anhydrous triethyl orthoacetate (2.51 mL, 13.68 mmol, 4.0 equiv.) in one portion via syringe, followed by a catalytic amount of pyridinium para-tolyl sulfonate (PPTS, 86 mg, 0.34 mmol, 10 mol%), at ambient temperature under a stream of dry argon. The reaction mixture was stirred at ambient temperature under an argon atmosphere until the solid dissolved completely and the solution became homogeneous (about ≈ 30 min). The resulting colourless solution was heated gently at 50-55 °C (caution, do not heat beyond 60 °C, risk of epimerization at C2 carbon) for about ≈ 4 h, until TLC analysis (silica gel, n-hexane/EtOAc 7:3) showed that the reaction was complete (the reaction mixture turned pale-yellow in colour at this stage). The crude reaction medium was diluted with water and ethyl acetate, the layers were separated, and the aqueous layer was extracted twice with ethyl acetate. The combined organic layers were washed once with water and once with saturated brine solution, dried over anhydrous Na2SO4, filtered and concentrated under reduced pressure. The crude product (yellow oil) was purified by column chromatography on silica gel employing an elution gradient of ethyl acetate in n-hexane, affording the desired pure (2R,3S)-5,7-di-O-benzyl-3'-O-methyl 3-acetylcatechin as a white amorphous solid. Isolated yield 88% (1.58 g, 3.0 mmol). Rf = 0.30 (silica gel, n-hexane/EtOAc 7:3). 1H-NMR (400 MHz, CDC13): δ 7.30-7.44 (10H, m, benzylic CarH), 6.86-6.89 (3H, m, CarH), 6.27 (2H, s, A2 spin-system, CarH), 5.37 (1H, q, J = 7.0 Hz, CH2-CH-OAc), 5.01 (4H, s, 2 x benzylic CH2), 4.99 (1H, d, Jtrans = 7.0 Hz, Car-CH-O), 3.85 (3H, s, methyloxy CH3), 2.98 (1H, dd, J1 = 16.8 Hz, J2 = 5.6 Hz, diastereotopic CH2, A part of AB spin-system), 2.74 (1H, dd, J1 = 16.8 Hz, J2 = 7.0 Hz, diastereotopic CH2, B part of AB spin-system), 1.95 (3H, s, acetyl CH3) ppm. 13C-NMR (100 MHz, CDC13): δ 170.2 (acetyl C=O), 159.1 (Cq), 157.8 (Cq), 155.1 (Cq), 146.7 (Cq), 145.8 (Cq), 137.0 (Cq), 136.9 (Cq), 129.8 (Cq), 128.74 (CH), 128.69 (CH), 128.1 (CH), 128.0 (CH), 127.7 (CH), 127.4 (CH), 127.3 (CH), 120.3 (CH), 114.3 (CH), 109.0 (CH), 101.6 (Cq), 94.6 (CH), 94.0 (CH), 78.7 (CH-O), 70.3 (benzylic CH2), 70.1 (benzylic CH2), 69.2 (CH-O), 56.1 (methyloxy CH3), 24.5 (allylic CH2), 21.2 (acetyl CH3) ppm.

### Example 5b: Synthesis of (2R,3S)-5,7,3'-tri-O-benzyl-3-acetylcatechin.

The synthesis of the title compound is illustrated on Figure 12. It was performed following the procedures described in Example 5a, starting from (1S,2S)-1-(3-(benzyloxy)-4-hydroxyphenyl)-3-(2,4-bis(benzyloxy)-6-hydroxyphenyl)propane-1,2-diol.

Rf = 0.30 (silica gel, n-hexane/EtOAc 7:3). 1H-NMR (400 MHz, DMSO-d6): δ 9.12 (1H, s, phenolic OH), 7.26-7.44 (15H, m, benzylic CarH), 6.96 (1H, d, Jmeta = 1.6 Hz, CarH), 6.77 (1H, d, Jortho = 8.2 Hz, CarH), 6.74 (1H, dd, Jortho = 8.2 Hz, Jmeta = 1.6 Hz, CarH), 6.37 (1H, d, Jmeta = 2.2 Hz, CarH), 6.21 (1H, d, Jmeta = 2.2 Hz, CarH), 5.23 (1H, q, J = 6.0 Hz, CH2-CH-OAc), 5.08 (1H, d, J = 6.0 Hz, Car-CH-O), 5.07 (2H, s, benzylic CH2), 5.06 (2H, s, benzylic CH2), 5.03 (2H, s, benzylic CH2), 2.68 (1H, dd, J1 = 16.9 Hz, J2 = 5.4 Hz, diastereotopic CH2, A part of AB spin-system), 2.60 (1H, dd, J1 = 16.9 Hz, J2 = 6.2 Hz, diastereotopic CH2, B part of AB spin-system), 1.88 (3H, s, acetyl CH3) ppm. 13C-NMR (100 MHz, DMSO-d6): δ 169.5 (acetyl C=O), 158.3 (Cq), 157.1 (Cq), 154.4 (Cq), 146.9 (Cq), 146.2 (Cq), 137.1 (Cq), 137.0 (Cq), 136.9 (Cq), 128.4 (2 x CH), 128.3 (2 x CH), 128.2 (Cq), 127.8 (CH), 127.6 (2 x CH), 127.4 (CH), 119.5 (CH), 115.7 (CH), 112.8 (CH), 100.5 (Cq), 94.5 (CH), 93.6 (CH), 77.2 (CH-O), 70.0 (benzylic CH2), 69.3 (benzylic CH2), 69.2 (benzylic CH2), 68.2 (CH-O), 23.2 (allylic CH2), 20.8 (acetyl CH3) ppm.

### Example 5c: Synthesis of (2R,3S)-5,7,4'-tri-O-benzyl-3-acetylcatechin.

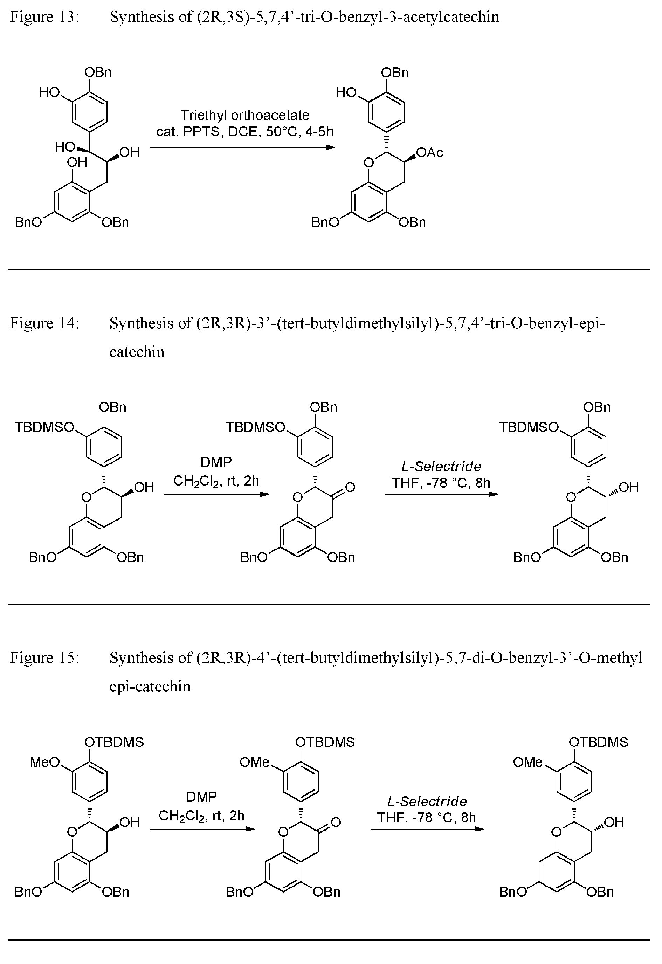

The synthesis of the title compound is illustrated on Figure 13. It was performed following the procedures described in Example 5a, starting from (1S,2S)-1-(4-(benzyloxy)-3-hydroxyphenyl)-3-(2,4-bis(benzyloxy)-6-hydroxyphenyl)propane-1,2-diol.

Rf = 0.26 (silica gel, n-hexane/EtOAc 7:3). 1H-NMR (400 MHz, DMSO-d6): δ 9.10 (1H, s, phenolic OH), 7.26-7.43 (15H, m, benzylic CarH), 6.91 (1H, d, Jortho = 8.4 Hz, CarH), 6.76 (1H, d, Jmeta = 2.0 Hz, CarH), 6.64 (1H, dd, Jortho = 8.4 Hz, Jmeta = 2.0 Hz, CarH), 6.34 (1H, d, Jmeta = 2.3 Hz, CarH), 6.21 (1H, d, Jmeta = 2.3 Hz, CarH), 5.17 (1H, q, J = 5.6 Hz, CH2-CH-OAc), 5.05 (1H, d, J = 5.6 Hz, Car-CH-O), 5.04 (2H, s, benzylic CH2), 5.03 (2H, s, benzylic CH2), 5.02 (2H, s, benzylic CH2), 2.61-2.63 (2H, m, diastereotopic CH2), 1.89 (3H, s, acetyl CH3) ppm. 13C-NMR (100 MHz, DMSO-d6): δ 169.6 (acetyl C=O), 158.4 (Cq), 157.1 (Cq), 154.3 (Cq), 146.8 (Cq), 146.3 (Cq), 137.2 (Cq), 137.0 (Cq), 136.9 (Cq), 130.7 (Cq), 128.42 (CH), 128.4 (CH), 128.3 (CH), 127.8 (CH), 127.76 (CH), 127.7 (CH), 127.67 (CH), 127.6 (CH), 127.4 (CH), 117.0 (CH), 114.0 (CH), 113.6 (CH), 100.4 (Cq), 94.4 (CH), 93.5 (CH), 76.9 (CH-O), 69.8 (benzylic CH2), 69.3 (2 x benzylic CH2), 68.2 (CH-O), 22.8 (allylic CH2), 20.8 (acetyl CH3) ppm.

### Example 6

### Inversion of Configuration at C3

### Example 6a: Synthesis of (2R,3R)-3'-(tert-butyldimethylsilyl)-5,7,4'-tri-O-benzyl-epi-catechin.

The synthesis of the title compound is illustrated on Figure 14.

### (2R)-3'-(tert-butyldimethylsilyl)-5,7,4'-tri-O-benzylflavan-3-one

The Dess-Martin oxidation of (2R,3S)-3'-(tert-butyldimethylsilyl)-5,7,4'-tri-O-benzylcatechin was carried out under mild reaction conditions, according to an experimental procedure adapted from the works of Chan et al. (Bioorg. Med. Chem. 2004, 12 (13), 3521-3527) and Barron et al. (Eur. J. Org. Chem. 2008, (36), 6069-6078). Accordingly, Dess-Martin periodinane (0.3 M solution in CH2Cl2, 8.3 mL, 2.48 mmol, 2.0 equiv.) was added in one portion to a well-stirred solution of (2R,3S)-3'-(tert-butyldimethylsilyl)-5,7,4'-tri-O-benzylcatechin (835 mg, 1.24 mmol, 1.0 equiv.) in anhydrous methylene chloride (40 mL) inside a flame-dried two-necked, 100 mL round-bottomed flask. The resulting pale-yellow solution was stirred at ambient temperature under an argon atmosphere for about ≈ 2 h. The crude residue (yellow-orange oil) was quickly purified by filtration through a pre-packed silica gel cartridge, employing an elution gradient of ethyl acetate in n-hexane, affording the desired pure (2R)-3'-(tert-butyldimethylsilyl)-5,7,4'-tri-O-benzylflavan-3-one as a pale-yellow oil. Isolated yield 75% (346 mg, 0.51 mmol). Rf = 0.39 (silica gel, n-hexane/EtOAc 4:1). 1H-NMR (400 MHz, CDCl3): δ 7.28-7.44 (15H, m, benzylic CarH), 6.85-6.87 (3H, m, CarH), 6.38 (1H, d, Jmeta = 2.2 Hz, CarH), 6.33 (1H, d, Jmeta = 2.2 Hz, CarH), 5.27 (1H, broad s, Car-CH-O), 5.02 (4H, s, 2 x benzylic CH2), 5.01 (2H, s, benzylic CH2), 3.60 (1H, dd, J1 = 21.6 Hz, J2 = 0.6 Hz, diastereotopic CH2, A part of AB spin-system), 3.52 (1H, d, J = 21.6 Hz, diastereotopic CH2, B part of AB spin-system), 0.93 (9H, s, Si-C(CH3)3), 0.06 (6H, s, 2 x Si-CH3) ppm. 13C-NMR (100 MHz, CDCl3): δ 205.0 (ketonic Cq), 159.5 (Cq), 157.1 (Cq), 154.5 (Cq), 150.5 (Cq), 145.5 (Cq), 136.9 (Cq), 136.6 (Cq), 136.5 (Cq), 128.6 (CH), 128.5 (CH), 128.4 (CH), 128.1 (Cq), 128.0 (CH), 127.9 (CH), 127.7 (2 x CH), 127.6 (CH), 127.2 (CH), 120.0 (CH), 119.4 (CH), 114.0 (CH), 102.0 (Cq), 95.9 (CH), 95.0 (CH), 82.9 (CH-O), 70.8 (benzylic CH2), 70.3 (benzylic CH2), 70.1 (benzylic CH2), 33.6 (α-carbonyl CH2), 25.7 (Si-C(CH3)3), 18.3 (Si-C(CH3)3), -4.6 (2 x Si-CH3) ppm.

### (2R,3R)-3'-(tert-butyldimethylsilyl)-5,7,4'-tri-O-benzyl-epi-catechin

The diastereoselective reduction of (2R)-3'-(tert-butyldimethylsilyl)-5,7,4'-tri-O-benzylflavan-3-one was carried out inside a flame-dried 50 mL two-necked round-bottomed flask, equipped with a magnetic stirrer bar and capped with a rubber septum, according to an experimental procedure adapted from the works of Chan et al. (Bioorg. Med. Chem. 2004, 12 (13), 3521-3527) and Barron et al. (Eur. J. Org. Chem. 2008, (36), 6069-6078). Accordingly, under an atmosphere of dry argon, (2R)-3'-(tert-butyldimethylsilyl)-5,7,4'-tri-O-benzylflavan-3-one (590 mg, 0.88 mmol, 1.0 equiv.) was dissolved in anhydrous tetrahydrofuran (20 mL), and the resulting colourless solution was cooled down to -78 °C (dry ice/acetone bath). Then, L-Selectride (1.0 M solution in THF, 1.76 mL, 1.76 mmol, 2.0 equiv.) was added dropwise via syringe. The resulting orange-yellow solution was stirred under an argon atmosphere at -78 °C for about ≈ 8 h. After quenching and subsequent work-up, the crude residue was purified by column chromatography on silica gel employing an elution gradient of ethyl acetate in n-hexane, affording the desired pure (2R,3R)-3'-(tert-butyldimethylsilyl)-5,7,4'-tri-O-benzyl-epi-catechin as an orange oil. Isolated yield 75% (258 mg, 0.38 mmol). Rf = 0.62 (silica gel, n-hexane/EtOAc 7:3). 1H-NMR (400 MHz, DMSO-d6): δ 7.31-7.47 (15H, m, benzylic CarH), 7.02 (1H, d, Jortho = 8.3 Hz, CarH), 6.97 (1H, s, CarH), 6.95 (1H, d, Jmeta = 1.8 Hz, CarH), 6.32 (1H, d, Jmeta = 2.3 Hz, CarH), 6.15 (1H, d, Jmeta = 2.3 Hz, CarH), 5.07 (2H, s, benzylic CH2), 5.06 (2H, s, benzylic CH2), 5.05 (2H, s, benzylic CH2), 4.91 (1H, s, hydroxylic OH), 4.85 (1H, d, J = 4.5 Hz, Car-CH-O), 4.06-4.10 (1H, m, CH2-CH-OH), 2.81 (1H, dd, J1 = 16.6 Hz, J2 = 4.5 Hz, diastereotopic CH2, A part of AB spin-system), 2.57 (1H, dd, J1 = 16.6 Hz, J2 = 3.9 Hz, diastereotopic CH2, B part of AB spin-system), 0.90 (9H, s, Si-C(CH3)3), 0.04 (6H, s, 2 x Si-CH3) ppm. 13C-NMR (100 MHz, DMSO-d6): δ 157.9 (Cq), 157.5 (Cq), 155.4 (Cq), 148.8 (Cq), 143.7 (Cq), 137.3 (Cq), 137.2 (Cq), 137.0 (Cq), 132.0 (Cq), 128.4 (CH), 128.3 (CH), 128.2 (CH), 127.8 (CH), 127.7 (CH), 127.6 (CH), 127.5 (CH), 127.2 (CH), 120.5 (CH), 119.6 (CH), 113.1 (CH), 101.6 (Cq), 94.5 (CH), 93.2 (CH), 77.8 (CH-O), 69.9 (benzylic CH2), 69.2 (benzylic CH2), 69.1 (benzylic CH2), 64.3 (CH-O), 28.0 (allylic CH2), 25.5 (Si-C(CH3)3), 18.0 (Si-C(CH3)3), -4.7 (Si-CH3) ppm.

### Example 6b: Synthesis of (2R,3R)-4'-(tert-butyldimethylsilyl)-5,7-di-O-benzyl-3'-O-methyl epi-catechin.

The synthesis of the title compound is illustrated on Figure 15. It was performed following the procedures described in Example 6a, starting from (2R,3S)-4'-(tert-butyldimethylsilyl)-5,7-di-O-benzyl-3'-O-methyl catechin.

Rf = 0.33 (silica gel, n-hexane/EtOAc 4:1). 1H-NMR (400 MHz, CDCl3): δ 7.30-7.43 (10H, m, benzylic CarH), 7.05 (1H, d, Jmeta = 1.9 Hz, CarH), 6.94 (1H, dd, Jlortho = 8.2 Hz, J2meta = 2.0 Hz, CarH), 6.89 (1H, d, Jortho = 8.2 Hz, CarH), 6.31 (1H, d, Jmeta = 2.3 Hz, CarH), 6.29 (1H, d, Jmeta = 2.3 Hz, CarH), 5.03 (2H, s, benzylic CH2), 5.02 (2H, s, benzylic CH2), 4.96 (1H, s, Car-CH-O), 4.26-4.28 (1H, m, CH2-CH-OH), 3.84 (3H, s, methyloxy CH3), 3.04 (1H, dd, J1 = 17.3 Hz, J2 = 1.6 Hz, diastereotopic CH2, A part of AB spin-system), 2.95 (1H, dd, J1 = 17.3 Hz, J2 = 4.4 Hz, diastereotopic CH2, B part of AB spin-system), 1.74 (1H, d, J = 5.3 Hz, hydroxylic OH), 1.01 (9H, s, Si-C(CH3)3), 0.17 (6H, s, 2 x Si-CH3) ppm. 13C-NMR (100 MHz, CDCl3 δ 158.9 (Cq), 158.5 (Cq), 155.5 (Cq), 151.3 (Cq), 145.2 (Cq), 137.2 (Cq), 137.1 (Cq), 131.7 (Cq), 128.74 (CH), 128.68 (CH), 128.1 (CH), 128.0 (CH), 127.7 (CH), 127.4 (CH), 127.3 (CH), 121.1 (CH), 118.9 (CH), 110.6 (CH), 101.2 (Cq), 94.9 (CH), 94.3 (CH), 78.7 (CH-O), 70.3 (benzylic CH2), 70.1 (benzylic CH2), 66.6 (CH-O), 55.8 (methyloxy CH3), 28.3 (allylic CH2), 25.9 (Si-C(CH3)3), 18.6 (Si-C(CH3)3), - 4.6 (2 x Si-CH3) ppm.

### Example 7

### Conjugation of the Protected (Epi)catechins

### Example 7a: Synthesis of (2R,3S)-catechin 4'-β-D-glucuronic acid.

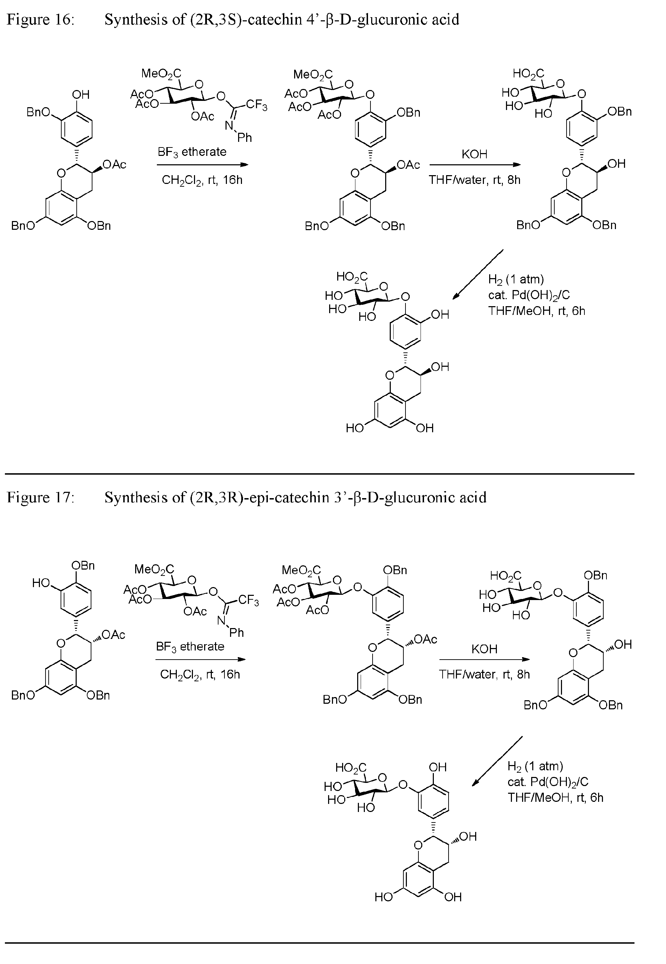

The synthesis of the title compound is illustrated on Figure 16.

### (2R,3S)-5,7,3'-tri-O-benzyl-3-acetylcatechin 4'-(2,3,4-tri-O-acetyl-β-D-methylglucuronate)

The glucuronidation reaction of (2R,3S)-5,7,3'-tri-O-benzyl-3-acetylcatechin was carried out under mild conditions in presence of BF3 etherate as catalyst, following an experimental procedure adapted from the works of Barron et al. (Org. Biomol. Chem. 2010, 8 (22), 5199-5211). Accordingly, to a well-stirred solution of (2R,3S)-5,7,3'-tri-O-benzyl-3-acetylcatechin (400 mg, 0.66 mmol, 1.0 equiv.) and glucuronic acid donor (536 mg, 1.06 mmol, 1.6 equiv.) in anhydrous methylene chloride (8.5 mL) was added catalytic boron trifluoride ethyl etherate (BF3_{·}OEt2, 23 µL, 0.066 mmol, 10 mol%) in one portion via microsyringe at 0 °C (ice/water bath). The resulting purple solution was stirred at 0 °C for 1 h (during this period, the colour of the reaction medium changed from purple to orange), followed by stirring at ambient temperature under an argon atmosphere for overnight (about ≈ 16 h). Upon completion (monitoring by TLC on silica gel, n-hexane/EtOAc 3:2), the crude reaction mixture was diluted with ethyl acetate and water and the layers were separated. The aqueous layer was extracted twice with ethyl acetate, and the combined organic layers were washed once with water and once with saturated brine solution, then dried over anhydrous Na2SO4, filtered and concentrated under reduced pressure. The resulting yellow oil was purified by column chromatography on silica gel employing an elution gradient of ethyl acetate in n-hexane, affording the desired pure (2R,3S)-5,7,3'-tri-O-benzyl-3-acetylcatechin 4'-(2,3,4-tri-O-acetyl-β-D-methylglucuronate) as a white amorphous solid. Isolated yield 92% (555 mg, 0.60 mmol). Rf = 0.14 (silica gel, n-hexane/EtOAc 3:2). 1H-NMR (400 MHz, DMSO-d6): δ 7.27-7.45 (15H, m, benzylic CarH), 7.10 (1H, d, Jmeta = 1.7 Hz, CarH), 7.07 (1H, dd, J1ortho = 8.4 Hz, J2meta = 1.7 Hz, CarH), 6.88 (1H, dd, Jlortho = 8.4 Hz, J2meta = 1.7 Hz, CarH), 6.38 (1H, d, Jmeta = 2.2 Hz, CarH), 6.24 (1H, d, Jmeta = 2.2 Hz, CarH), 5.54 (1H, d, Jax-ax = 7.9 Hz, sugar anomeric CH), 5.42-5.48 (1H, m, sugar CH), 5.28 (1H, broad q, J = 5.8 Hz, CH2-CH-OAc), 5.11-5.15 (2H, m, sugar CH), 5.09 (1H, d, J = 5.6 Hz, Car-CH-O), 5.07 (2H, s, benzylic CH2), 5.06 (2H, s, benzylic CH2), 5.04 (2H, AB q, J = 11.3 Hz, diastereotopic benzylic CH2), 4.65 (1H, d, Jax-ax = 9.9 Hz, sugar α-carbonyl CH), 3.63 (3H, s, methyl ester CH3), 2.58-2.69 (2H, m, diastereotopic CH2), 2.00 (3H, s, acetyl CH3), 1.99 (3H, s, acetyl CH3), 1.91 (3H, s, acetyl CH3), 1.81 (3H, s, acetyl CH3) ppm. 13C-NMR (100 MHz, DMSO-d6): δ 169.5 (acetyl C=O), 169.4 (acetyl C=O), 169.2 (acetyl C=O), 168.8 (acetyl C=O), 167.1 (methyl ester C=O), 158.4 (Cq), 157.1 (Cq), 154.1 (Cq), 148.3 (Cq), 145.5 (Cq), 137.0 (Cq), 136.9 (Cq), 136.7 (Cq), 133.6 (Cq), 128.41 (CH), 128.40 (CH), 128.39 (CH), 128.2 (CH), 127.8 (CH), 127.7 (CH), 127.4 (CH), 127.3 (CH), 127.2 (CH), 119.0 (CH), 117.8 (CH), 113.1 (CH), 100.4 (Cq), 98.1 (sugar anomeric CH), 94.5 (CH), 93.7 (CH), 76.7 (CH-O), 71.0 (sugar CH), 70.9 (sugar CH), 70.4 (sugar CH), 69.9 (benzylic CH2), 69.3 (benzylic CH2), 69.2 (benzylic CH2), 69.0 (sugar CH), 68.0 (CH-O), 52.5 (methyl ester CH3), 23.0 (allylic CH2), 20.8 (acetyl CH3), 20.3 (acetyl CH3), 20.2 (acetyl CH3), 20.1 (acetyl CH3) ppm.

### (2R,3S)-5,7,3'-tri-O-benzylcatechin 4'-β-D-glucuronic acid

The alkaline hydrolysis of (2R,3S)-5,7,3'-tri-O-benzyl-3-acetylcatechin 4'-(2,3,4-tri-O-acetyl-β-D-methylglucuronate) was carried out with KOH in a solvent mixture of THF and water, following an experimental procedure adapted from the works of Yu et al. (J. Org. Chem. 2005, 70 (22), 8884-8889). Accordingly, to a well-stirred solution of (2R,3S)-5,7,3'-tri-O-benzyl-3-acetylcatechin 4'-(2,3,4-tri-O-acetyl-β-D-methylglucuronate) (550 mg, 0.59 mmol, 1.0 equiv.) in THF/water (4:1 v/v) was added potassium hydroxide (KOH, anhydrous pellets, 463 mg, 8.3 mmol, 14 equiv.) at ambient temperature under an argon atmosphere. After stirring at ambient temperature for about ≈ 8 h (monitoring by TLC on RP-18 phase, water/MeCN 1:1), the reaction mixture was neutralized by the addition of Amberlite IRC 50 ion-exchange resin, and then adjusted to pH ≈ 6 by adding a few drops of formic acid. The resulting suspension was filtered (washed with methanol), the filtrate was evaporated to dryness under reduced pressure and re-dissolved in water. The obtained aqueous solution was lyophilized, yielding crude (2R,3S)-5,7,3'-tri-O-benzylcatechin 4'-β-D-glucuronic acid. The crude residue was purified by column chromatography on RP-18 phase employing an elution gradient of acetonitrile in water, affording the desired pure (2R,3S)-5,7,3'-tri-O-benzylcatechin 4'-β-D-glucuronic acid as a white amorphous solid. Quantitative yield (436 mg, 0.59 mmol). Rf = 0.43 (silica gel, CH2Cl2/MeOH 3:2). Rf = 0.27 (RP-18 phase, water/MeCN 1:1). 1H-NMR (400 MHz, DMSO-d6): δ 12.72 (1H, broad s, acidic CO2H), 7.26-7.48 (15H, m, benzylic CarH), 7.11 (1H, d, Jortho = 8.5 Hz, CarH), 7.05 (1H, d, Jmeta = 1.6 Hz, CarH), 6.90 (1H, dd, Jlortho = 8.5 Hz, J2meta = 1.6 Hz, CarH), 6.33 (1H, d, Jmeta = 2.1 Hz, CarH), 6.15 (1H, d, Jmeta = 2.1 Hz, CarH), 5.17 (1H, broad s, hydroxylic OH), 5.10 (2H, s, benzylic CH2), 5.08 (2H, s, benzylic CH2), 5.04 (2H, s, benzylic CH2), 5.02-5.11 (2H, broad s, hydroxylic OH), 4.94 (1H, d, J = 7.0 Hz, Car-CH-O), 4.65 (1H, d, Jax-ax = 7.5 Hz, sugar anomeric CH), 3.94 (1H, quin., J = 5.6 Hz, CH2-CH-OH), 3.46 (1H, d, Jax-ax = 9.6 Hz, sugar α-carbonyl CH), 3.28 (1H, broad s, hydroxylic OH), 3.18-3.26 (3H, m, sugar CH), 2.76 (1H, dd, J1 = 16.7 Hz, J2 = 5.4 Hz, diastereotopic CH2, A part of AB spin-system), 2.47 (1H, dd, J1 = 16.7 Hz, J2 = 8.0 Hz, diastereotopic CH2, B part of AB spin-system) ppm. 13C-NMR (100 MHz, DMSO-d6): δ 171.5 (acidic CO2H), 158.0 (Cq), 157.1 (Cq), 155.0 (Cq), 147.7 (Cq), 147.0 (Cq), 137.4 (Cq), 137.2 (Cq), 137.1 (Cq), 133.0 (Cq), 128.4 (CH), 128.36 (CH), 128.2 (CH), 127.7 (CH), 127.68 (CH), 127.6 (CH), 127.58 (CH), 127.5 (CH), 127.2 (CH), 120.3 (CH), 116.0 (CH), 114.9 (CH), 102.2 (Cq), 100.3 (sugar anomeric CH), 94.3 (CH), 93.4 (CH), 80.9 (CH-O), 76.8 (sugar CH), 74.0 (sugar CH), 73.2 (sugar CH), 71.9 (sugar CH), 70.6 (benzylic CH2), 69.2 (2 x benzylic CH2), 65.7 (CH-O), 27.9 (allylic CH2) ppm.

### (2R,3S)-catechin 4'-β-D-glucuronic acid

The title compound was prepared according to a reductive de-benzylation protocol adapted from the works of Chan et al. (Bioorg. Med. Chem. 2005, 13 (6), 2177-2185). Accordingly, palladium (II) hydroxide over activated charcoal (Pd(OH)2/C ≈ 15-20%, 650 mg, 1764 g/mol) was added in one portion to a well-stirred suspension of (2R,3S)-5,7,3'-tri-O-benzylcatechin 4'-β-D-glucuronic acid (196 mg, 0.27 mmol, 1.0 equiv.) in a solvent mixture of THF/MeOH (1:1 v/v, 32 mL). After a few evacuation/H2 refilling cycles, the reaction mixture was stirred at ambient temperature under an H2 atmosphere (1 atm) for 6 h when TLC analysis on RP-18 coated silica plates (eluting with water/CH3CN 95:5 + 1% formic acid) showed that the reaction was completed. The crude reaction mixture was then filtered through a sintered glass funnel fitted with a glass fiber prefilter, washed with methanol. The filtrate was evaporated to dryness under reduced pressure, and the crude residue was rapidly purified by preparative HPLC chromatography on a phenyl-hexyl column employing an elution gradient of methanol in water (0.1% formic acid), and subsequently freeze-dried, affording the desired pure (2R,3S)-catechin 4'-β-D-glucuronic acid as a white amorphous solid. Isolated yield 78% (100 mg, 0.21 mmol). Rf = 0.76 (RP-18 phase, water/MeCN 4:1 + 1% formic acid). 1H-NMR (400 MHz, methanol-d4): δ 7.13 (1H, d, Jortho = 8.3 Hz, CarH), 6.92 (1H, d, Jmeta = 1.8 Hz, CarH), 6.84 (1H, dd, Jlortho = 8.3 Hz, J2meta = 1.8 Hz, CarH), 5.94 (1H, d, Jmeta = 2.3 Hz, CarH), 5.87 (1H, d, Jmeta = 2.3 Hz, CarH), 4.86 (9H, broad s, hydroxylic OH + sugar anomeric CH), 4.61 (1H, d, J = 7.6 Hz, Car-CH-O), 3.95-4.00 (1H, m, CH2-CH-OH), 3.93 (1H, d, Jax-ax = 9.6 Hz, sugar α-carbonyl CH), 3.47-3.64 (3H, m, sugar CH), 2.84 (1H, dd, J1 = 16.2 Hz, J2 = 5.5 Hz, diastereotopic CH2, A part of AB spin-system), 2.51 (1H, dd, J1 = 16.2 Hz, J2 = 8.2 Hz, diastereotopic CH2, B part of AB spin-system) ppm. 13C-NMR (100 MHz, methanol-d4): δ 172.4 (carbonyl CO2H), 157.9 (Cq), 157.6 (Cq), 156.8 (Cq), 148.5 (Cq), 146.3 (Cq), 136.8 (Cq), 119.9 (CH), 118.9 (CH), 116.2 (CH), 104.4 (sugar anomeric CH), 100.8 (Cq), 96.4 (CH), 95.5 (CH), 82.6 (CH-O), 77.1 (sugar CH), 76.7 (sugar CH), 74.6 (sugar CH), 73.1 (sugar CH), 68.9 (CH-O), 28.6 (allylic CH2) ppm. UPLC retention time tR 1.31 min. LC/ESI-TOF-MS (-) (m/z): calc. for C21H21O12 [M-H]- 465.1033, obs. 465.1027 (β-anomer), obs. 465.1013 (α-anomer). [α]D25 = -7.3 (c = 1 mg/mL, MeOH).

### Example 7b: Synthesis of (2R,3R)-epi-catechin 3'-β-D-glucuronic acid.

The synthesis of the title compound is illustrated on Figure 17. It was performed following the procedures described in Example 7a, starting from (2R,3R)-5,7,4'-tri-O-benzyl-3-acetyl-epi-catechin.

Rf = 0.23 (RP-18 phase, water/MeOH 4:1 + 1% formic acid). 1H-NMR (400 MHz, methanol-d4): δ 7.37 (1H, d, Jmeta = 1.8 Hz, CarH), 7.09 (1H, dd, Jlortho = 8.3 Hz, J2meta = 1.8 Hz, CarH), 6.85 (1H, d, Jortho = 8.3 Hz, CarH), 5.92-5.95 (2H, m, CarH), 4.86 (1H, d, Jax-ax = 6.8 Hz, sugar anomeric CH), 4.85 (8H, s, hydroxylic OH), 4.81 (1H, s, Car-CH-O), 4.21-4.23 (1H, m, CH2-CH-OH), 3.84 (1H, d, Jax-ax = 9.4 Hz, sugar α-carbonyl CH), 3.47-3.60 (3H, m, sugar CH), 2.87 (1H, dd, J1 = 16.8 Hz, J2 = 4.5 Hz, diastereotopic CH2, A part of AB spin-system), 2.75 (1H, dd, J1 = 16.8 Hz, J2 = 2.6 Hz, diastereotopic CH2, B part of AB spin-system) ppm. 13C-NMR (100 MHz, methanol-d4): δ 174.2 (carbonyl CO2H), 158.0 (Cq), 157.7 (Cq), 157.3 (Cq), 148.3 (Cq), 146.4 (Cq), 132.5 (Cq), 123.7 (CH), 118.6 (CH), 116.9 (CH), 104.8 (sugar anomeric CH), 100.1 (Cq), 96.5 (CH), 96.0 (CH), 79.6 (CH-O), 77.3 (sugar CH), 76.6 (sugar CH), 74.7 (sugar CH), 73.3 (sugar CH), 67.3 (CH-O), 29.3 (allylic CH2) ppm. UPLC retention time tR 3.45 min (β-anomer), 3.71 min (α-anomer). LC/ESI-TOF-MS (-) (m/z): calc. for C21H21O12 [M-H]- 465.1033, obs. 465.1048 (β-anomer), obs. 465.0889 (α-anomer). [α]D25 = -6.6 (c = 1 mg/mL, MeOH).

### Example 7c: Synthesis of (2R,3R)-3'-O-methyl epicatechin 4'-sodium sulfate.

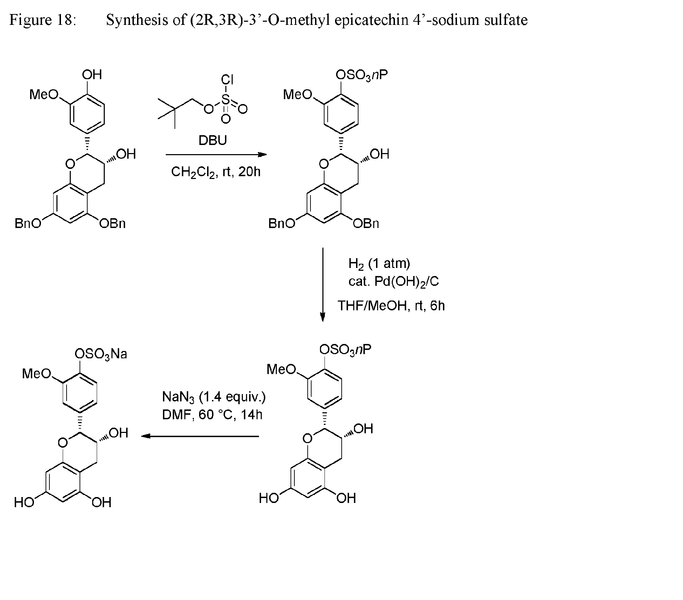

The synthesis of the title compound is illustrated on Figure 18.

### (2R,3R)-5,7-di-O-benzyl-3'-O-methyl epi-catechin 4'-neopentylsulfate

The regioselective mono-sulfation reaction of (2R,3R)-5,7-di-O-benzyl-3'-O-methyl epicatechin was carried out inside a flame-dried 25 mL two-necked round-bottomed flask equipped with a magnetic stirrer bar and capped with a rubber septum, according to an experimental procedure adapted from the works of Widlanski et al. (J. Am. Chem. Soc. 2006, 128 (5), 1605-1610). Accordingly, (2R,3R)-5,7-di-O-benzyl-3'-O-methyl epi-catechin (200 mg, 0.41 mmol, 1.0 equiv.) was dissolved in anhydrous methylene chloride (5 mL) and the resulting colourless solution was equilibrated at room temperature under an argon atmosphere. Then, 1,8-diazabicycloundec-7-ene (DBU, 100 µL, 0.66 mmol, 1.6 equiv.) was introduced in one portion via syringe, and the resulting yellow solution was cooled down to 0 °C (ice/water bath). A solution of neopentyl chlorosulfate (124 mg, 0.66 mmol, 1.6 equiv.) in anhydrous methylene chloride (1.5 mL) was then added dropwise via syringe. After stirring at 0 °C for about 1 h, the resulting pink solution was warmed-up to ambient temperature and stirred at that temperature for overnight (≈ 20 h). Upon completion (monitoring by TLC on silica gel, n-hexane/EtOAc 3:2), the crude reaction medium was diluted with water and ethyl acetate, and the layers were separated. The aqueous layer was extracted twice with ethyl acetate. The combined organic layers were washed once with saturated NH4Cl aqueous solution and once with saturated brine solution, then dried over anhydrous Na2SO4, filtered and liberated of solvents in vacuo. The crude residue was purified by column chromatography on silica gel employing an elution gradient of ethyl acetate in n-hexane, affording the desired pure (2R,3R)-5,7,4'-tri-O-benzyl-epi-catechin 3'-neopentylsulfate as a colorless oil. Isolated yield 75% (197 mg, 0.31 mmol). Rf = 0.44 (silica gel, n-hexane/EtOAc 3:2). 1H-NMR (400 MHz, CDCl3): δ 7.31-7.43 (11H, m, CarH), 7.22 (1H, d, Jmeta = 1.6 Hz, CarH), 7.06 (1H, dd, Jlortho = 8.6 Hz, J2meta = 1.7 Hz, CarH), 6.30 (2H, s, CarH), 5.04 (2H, s, benzylic CH2), 5.02 (2H, s, benzylic CH2), 5.00 (1H, s, Car-CH-O), 4.28-4.31 (1H, m, CH2-CH-OH), 4.18 (2H, s, neopentyl CH2), 3.93 (3H, s, methyloxy CH3), 3.05 (1H, d, J = 17.4 Hz, diastereotopic CH2, A part of AB spin-system), 2.98 (1H, dd, J1 = 17.4 Hz, J2 = 4.0 Hz, diastereotopic CH2, B part of AB spin-system), 1.71 (1H, d, J = 6.4 Hz, hydroxylic OH), 1.02 (9H, s, neopentyl CH3) ppm.

### (2R,3R)-3'-O-methyl epi-catechin 4'-neopentylsulfate

The title compound was prepared according to a reductive de-benzylation protocol adapted from the works of Chan et al. (Bioorg. Med. Chem. 2005, 13 (6), 2177-2185). Accordingly, palladium (II) hydroxide over activated charcoal (Pd(OH)2/C ≈ 15-20%, 60 mg, 1180 g/mol) was added in one portion to a well-stirred solution of (2R,3R)-5,7-di-O-benzyl-3'-O-methyl epi-catechin 4'-neopentylsulfate (32 mg, 0.05 mmol, 1.0 equiv.) in a solvent mixture of THF/MeOH (1:1 v/v, 6 mL). After a few evacuation/H2 refilling cycles, the reaction mixture was stirred at ambient temperature under an H2 atmosphere (1 atm) for 6 h when TLC analysis on silica gel plates (eluting with CH2Cl2/MeOH 94:6) showed that the reaction was completed. The crude reaction mixture was then filtered through a sintered glass funnel fitted with a glass fiber prefilter, washed with methanol. The filtrate was evaporated to dryness under reduced pressure, and the crude residue was rapidly purified by column chromatography on silica gel employing an elution gradient of methanol in methylene chloride, affording the desired pure (2R,3R)-3'-O-methyl epi-catechin 4'-neopentylsulfate as a brown oil. Isolated yield 80% (18 mg, 0.04 mmol). Rf = 0.13 (silica gel, CH2Cl2/MeOH 94:6). 1H-NMR (400 MHz, CDCl3): δ 7.27 (1H, d, Jortho = 8.0 Hz, CarH), 7.11 (1H, d, Jmeta = 1.6 Hz, CarH), 6.93 (1H, dd, Jlortho = 8.0 Hz, J2meta = 1.6 Hz, CarH), 5.98 (1H, d, Jmeta = 2.3 Hz, CarH), 5.79 (1H, d, Jmeta = 2.3 Hz, CarH), 4.79 (1H, s, Car-CH-O), 4.16 (2H, s, neopentyl CH2), 4.01-4.13 (1H, m, CH2-CH-OH), 3.79 (3H, s, methyloxy CH3), 2.73 (3H, broad s, hydroxylic OH), 1.23-1.27 (2H, m, diastereotopic CH2), 0.98 (9H, s, neopentyl CH3) ppm.

### (2R,3R)-3'-O-methyl epicatechin 4'-sodium sulfate

The de-neopentylation reaction of (2R,3R)-3'-O-methyl epi-catechin 4'-neopentylsulfate was carried out following an experimental procedure adapted from the works of Widlanski et al.(J. Am. Chem. Soc. 2006, 128 (5), 1605-1610). Accordingly, inside a flame-dried 5 mL two-necked round-bottomed flask fitted with a reflux condenser, (2R,3R)-3'-O-methyl epicatechin 4'-neopentylsulfate (17 mg, 0.037 mmol, 1.0 equiv.) was dissolved in anhydrous DMF (200 µL). Sodium azide (3.4 mg, 0.052 mmol, 1.4 equiv.) was added as a solid in one portion, and the resulting yellow-orange solution was stirred and heated to 70 °C in an oil bath under an argon atmosphere for overnight. Removal of the solvent under high vacuum (10-2 mbar, 40 °C) and subsequent purification of the crude product by preparative HPLC chromatography on a phenyl-hexyl column employing an elution gradient of methanol in water (0.1% formic acid) afforded, after neutralization with 0.1 N aqueous NaOH and subsequent lyophilization, the desired (2R,3R)-3'-O-methyl epi-catechin 4'-sodiumsulfate (contaminated with sodium formate salt) as a white amorphous solid. De-salination was accomplished by filtration through a short Strata-X polymeric reversed phase solid phase extraction (SPE) cartridge. Salts were removed with pure water, and then the desired sodium sulfate was eluted with pure methanol. Isolated yield 82% (12 mg, 0.031 mmol). Rf = 0.51 (RP-18 phase, water/MeOH 94:6 + 1% formic acid). 1H-NMR (400 MHz, methanol-d4): δ 7.44 (1H, d, Jortho = 8.3 Hz, CarH), 7.23 (1H, d, Jmeta = 1.8 Hz, CarH), 7.01 (1H, dd, Jlortho = 8.3 Hz, J2meta = 1.8 Hz, CarH), 5.97 (1H, d, Jmeta = 2.3 Hz, CarH), 5.94 (1H, d, Jmeta = 2.3 Hz, CarH), 4.94 (1H, broad s, Car-CH-O), 4.87 (3H, s, hydroxylic OH), 4.22-4.24 (1H, m, CH2-CH-OH), 3.87 (3H, s, methyloxy CH3), 2.90 (1H, dd, J1 = 16.8 Hz, J2 = 4.6 Hz, diastereotopic CH2, A part of AB spin-system), 2.76 (1H, dd, J1 = 16.8 Hz, J2 = 2.7 Hz, diastereotopic CH2, B part of AB spin-system) ppm. 13C-NMR (100 MHz, methanol-d4): δ 158.1 (Cq), 157.8 (Cq), 157.2 (Cq), 152.8 (Cq), 142.2 (Cq), 138.5 (Cq), 123.2 (CH), 119.8 (CH), 113.0 (CH), 100.1 (Cq), 96.6 (CH), 95.9 (CH), 79.7 (CH-O), 67.6 (CH-O), 56.7 (methyloxy CH3), 29.3 (allylic CH2) ppm. UPLC retention time tR 8.76 min. LC/ESI-TOF-MS (-) (m/z): calc. for C16H15O9S [M-Na]- 383.0436, obs. 383.0473. [α]D25 = -13 (c = 1 mg/mL, MeOH).

### Example 7d: Synthesis of (2R,3R)-epi-catechin 3'-sodium sulfate.

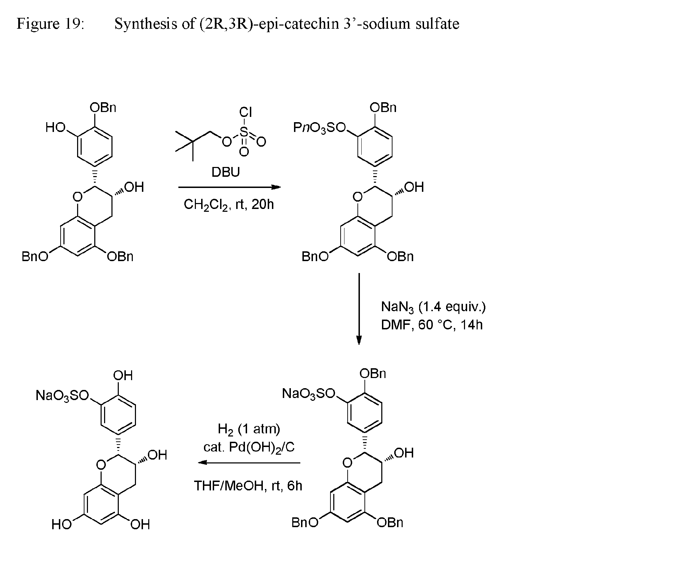

The synthesis of the title compound is illustrated on Figure 19. It was performed following the procedures described in Example 7c, starting from (2R,3R)-5,7,4'-tri-O-benzyl-epi-catechin. UPLC retention time tR 6.27 min. LC/ESI-TOF-MS (-) (m/z): calc. for C15H13O9S [M-Na]-369.0280, obs. 369.0291.

## Claims

1. Process for the preparation of a compound of the general formula (I) 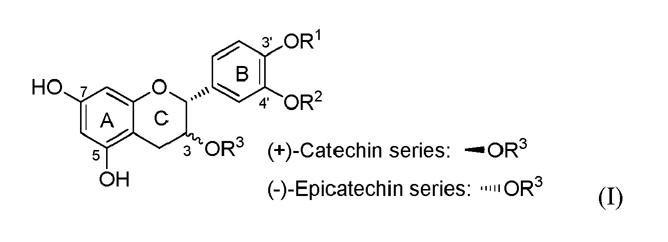 , wherein R¹ and R² are independently H, methyl, sulfonyl, glucuronyl or glucoside; and R³ is H or a gallate;
which comprises
a) a coupling reaction step of a compound of formula (II) 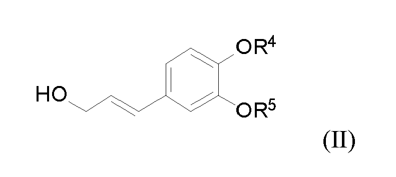 , wherein R⁴ and R⁵ are independently H, methyl or a protecting group; with a compound of formula (III) 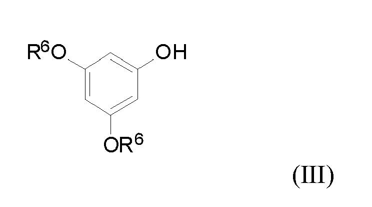 , wherein R⁶ is a protecting group; and
b) a stereo-selective intra-molecular trans-cyclization reaction step of the coupled product of step a).

2. The process of claim 1, wherein for the compound of the formula (II) R⁴ is not equal R⁵.

3. The process of claim 1 or 2 further comprising a Sharpless dihydroxylation reaction step after step a) and before step b).

4. The process of one of the above claims further comprising a step of inversion of the configuration at position C3 of the cyclized product of step b).

5. The process of one of the above claims, wherein the trans-cyclization reaction step b) is performed first in the presence of triethylorthoacetate and then followed by a deacetylation reaction at position C3 under reductive conditions.

6. The process of one of the above claims, wherein the protecting group of the compounds of the formula (II) and/or (III) is selected from the group consisting of benzyl and TBDMS.

7. The process of one of the above claims further comprising a reaction step of conjugating the cyclized product of step b) or said product after the inversion of the configuration as of claim 4, with a sulfate donor, a glucuronic acid donor and/or a glucose donor.

8. The process of claim 7, wherein the sulfate donor is selected from neopentylchlorosulfate or trichloroethylchlorosulfate, the glucuronic acid donor is selected from 2,3,4-tri-O-acetyl- α-D-methylglucuronopyranosyl-1-(N-phenyl)-2,2,2-trifluoroacetimidate, 2,3,4-tri-*O*-acetyl- α-D-methylglucopyranosyl-1-(N-4-methoxyphenyl)-2,2,2-trifluoroacetimidate, 2,3,4-tri-O-acetyl-α-D-methylglucuronopyranosyl-1-0-(2,2,2-trichloroacetimidate) or acetobromo-α-D-glucuronic acid methyl ester, and the glucose donor is selected from 2,3,4,6-tetra-0-acetyl- α-D-glucopyranosyl-1-(N-phenyl)-2,2,2-trifluoroacetimidate, 2,3,4,6-tetra-(9-acetyl-α-D-glucuropyranosyl-1-O-(2,2,2-trichloroacetimidate) or α-acetobromoglucose.

9. Compound of the general formula (I) 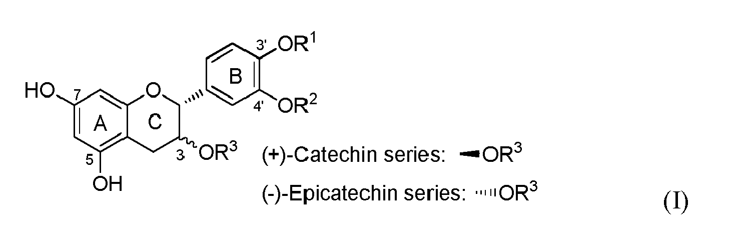 , wherein
R¹ and R² are independently methyl, sulfonyl, glucuronyl or glucoside, but are not both glucuronyl and/or glucoside;
R³ is H or a gallate; and
wherein the compound is not 4'-O-methyl-(-)-epicatechin-3'-O-glucuronide.

10. The compound of claim 9, wherein R¹ is not equal R².

11. The compound of claim 9 or 10, wherein R¹ is not glucuronyl or glucoside when R² is sulfonyl, or vice versa.

12. The compound of claim 9 to 11, wherein the compound is selected from the group consisting of 4'-O-methyl-(-)-epicatechin-3'-sulfate; 3'-O-methyl-(-)-epicatechin-4'-sulfate; 4'-O-methyl-(-)-epicatechin-3'-O-β-D-glucuronide; 3'-O-methyl-(-)-epicatechin-4'-*O*-β-D-glucuronide; 3'-O-methyl-(-)-epicatechin-4'-O- β-D-glycoside; and 4'-O-methyl-(-)-epicatechin-3'-O-β-D-glycoside.

13. Food product comprising a compound according to the claims 9 to 12.

14. The food product of claim 13, wherein the compound is present in a concentration of at least 0.01 mg/g wt%, preferably of at least 0.1 mg/g wt%, more preferably of at least 1 mg/g wt%, even more preferably of 10 mg/g wt% of the total food product, or more.
